# EUROPÄISCHE PATENTSCHRIFT

(11) **EP 2 379 736 B1**
(45) Veröffentlichungstag und Bekanntmachung des Hinweises auf die Patenterteilung: **08.03.2017**
(21) Anmeldenummer: 09799633.4
(22) Anmeldetag: 22.12.2009
(51) Int. Cl.: G01N 33/74, C12Q 1/02

(54) **VERFAHREN ZUM NACHWEIS UND/ODER ZUR IDENTIFIZIERUNG HORMONELL WIRKSAMER SUBSTANZEN**
METHOD FOR VERIFYING AND/OR IDENTIFYING HORMONALLY EFFECTIVE SUBSTANCES
PROCÉDÉ DE CRIBLAGE ET/OU D'IDENTIFICATION DE SUBSTANCES À ACTIVITÉ HORMONALE

(30) Priorität: 22.12.2008 DE 102008064553
(43) Veröffentlichungstag der Anmeldung: 26.10.2011
(73) Patentinhaber: Technische Universität Dresden, 01069 Dresden (DE)
(72) Erfinder: OSTERMANN, Kai, 01187 Dresden (DE); GROß, Annett, 01069 Dresden (DE); ZIERAU, Oliver, 01097 Dresden (DE); DIEL, Patrick, 50259 Pullheim (DE); VOLLMER, Günter, 01705 Pesterwitz (DE); WOLF, Sylvi, 01307 Dresden (DE); RATAJ, Felicitas, 99096 Erfurt (DE); RÖDEL, Gerhard, 85757 Karlsfeld (DE)
(74) Vertreter: Kailuweit & Uhlemann Patentanwälte Partnerschaft mbB
(86) Internationale Anmeldenummer: PCT/EP2009/067756
(87) Internationale Veröffentlichungsnummer: WO 2010/072767

(56) Entgegenhaltungen:
- WO-A1-2009/000918
- US-A- 6 159 705
- US-B1- 6 555 325
- BOVEE T F H ET AL: "Inter-laboratory comparison of a yeast bioassay for the determination of estrogenic activity in biological samples" ANALYTICA CHIMICA ACTA, ELSEVIER, AMSTERDAM, NL, Bd. 637, Nr. 1-2, 14. Oktober 2008 (2008-10-14), Seiten 265-272, XP025976929 ISSN: 0003-2670 [gefunden am 2008-10-14]
- ZIERAU O ET AL: "Detection of anabolic steroid abuse using a yeast transactivation system" STEROIDS, ELSEVIER SCIENCE PUBLISHERS, NEW YORK, NY, US, Bd. 73, Nr. 11, 1. Oktober 2008 (2008-10-01), Seiten 1143-1147, XP022849965 ISSN: 0039-128X [gefunden am 2008-05-04]
- BROWN ANDREW J ET AL: "Functional coupling of mammalian receptors to the yeast mating pathway using novel yeast/mammalian G protein alpha-subunit chimeras", YEAST, vol. 16, no. 1, 15 January 2000 (2000-01-15), pages 11-22, ISSN: 0749-503X
- DOWELL S J ET AL: "Yeast assays for G-protein-coupled receptors.", RECEPTORS & CHANNELS 2002, vol. 8, no. 5-6, 2002, pages 343-352, ISSN: 1060-6823
- MINIC JASMINA ET AL: "Functional expression of olfactory receptors in yeast and development of a bioassay for odorant screening", FEBS JOURNAL, vol. 272, no. 2, January 2005 (2005-01), pages 524-537, ISSN: 1742-464X(print)
- PAUSCH M H: "G-protein-coupled receptors in Saccharomyces cerevisiae: high-throughput screening assays for drug discovery", TRENDS IN BIOTECHNOLOGY, ELSEVIER PUBLICATIONS, CAMBRIDGE, GB, vol. 15, no. 12, 1 December 1997 (1997-12-01), pages 487-494, XP004097426, ISSN: 0167-7799, DOI: 10.1016/S0167-7799(97)01119-0
- REILANDER H ET AL: "Production of G-protein-coupled receptors in yeast", CURRENT OPINION IN BIOTECHNOLOGY, LONDON, GB, vol. 9, no. 5, 1 October 1998 (1998-10-01) , pages 510-517, XP002393050, ISSN: 0958-1669, DOI: 10.1016/S0958-1669(98)80038-4
- RADHIKA VENKAT ET AL: "Chemical sensing of DNT by engineered olfactory yeast strain", NATURE CHEMICAL BIOLOGY, vol. 3, no. 6, June 2007 (2007-06), pages 325-330, ISSN: 1552-4450(print)

## Beschreibung

Die Erfindung betrifft ein einfaches Verfahren zum Nachweis und/oder zur Identifizierung hormonell wirksamer Substanzen unter Nutzung des Pheromonsystems von Hefen, Hefezellen zur Durchführung des Verfahrens und die Verwendung des Verfahrens bzw. der Hefezellen. Die Erfindung eignet sich auch zur Durchführung von Screenings, bei denen Substanzgemische und/oder Substanzbibliotheken schnell und kostengünstig auf die Anwesenheit von hormonell wirksamen Substanzen durchsucht werden.

Hormone spielen in höheren Organismen eine wichtige Rolle, da sie den Austausch von Informationen zwischen entfernten Zellen, Zellgruppen, Geweben oder Organen erlauben. Die Wirkung der Hormone als Botenstoffe beruht dabei nicht auf einer direkten Einwirkung auf die Substrate, wie dies bei Enzymen der Fall ist, sondern auf der Wechselwirkung mit einem spezifischen Hormonrezeptor, über den in der Zielzelle eine biologische Antwort vermittelt wird. Die Hormonwirkung wird also nicht allein durch das Hormon selbst, sondern auch durch die Reaktionsbereitschaft, z. B. den Rezeptorstatus der Zielzelle, bedingt. Daher können Hormone in verschiedenen Zielzellen unterschiedliche Reaktionen hervorrufen.

Hormone können ihren chemischen Eigenschaften nach in vier Gruppen eingeteilt werden: Proteo- bzw. Peptidhormone, Steroidhormone, Arachidonsäurederivate (Eicosanoide) und Aminosäurederivate. Man kann außerdem folgende drei Gruppen unterscheiden:
Glanduläre Hormone werden in Drüsen, neurosekretorische Hormone von den Nervenzellen produziert. Beide Gruppen werden anschließend über die Blutbahn zu den Zielzellen transportiert. Dies wird als endokriner Mechanismus bezeichnet.

Gewebshormone hingegen wirken lokal in dem Gewebe, in dem sie produziert wurden. Dies wird als parakriner Mechanismus bezeichnet.

Hormone finden sowohl in natürlicher als auch in synthetischer Form Verwendung in der Medizin und der Veterinärmedizin beim Ersatz von spezifischen Ausfallerscheinungen oder zur Erzielung eines spezifischen Zustandes, z.B. als Kontrazeptiva, Antiarthritika, Antidiabetika und Anabolika. Allerdings kommt es regelmäßig auch zum Missbrauch von Hormonen und hormonähnlichen Substanzen. So werden Hormone und hormonähnliche Substanzen im Leistungssport und mittlerweile auch im Breitensport illegal zur Leistungssteigerung eingesetzt (Doping). Ein Charakteristikum von Hormonen ist, dass sie in Konzentrationsbereichen wirken, die häufig deutlich niedriger sind als bei anderen Therapeutika, was ihren Nachweis erschwert und eine sehr sensitive Sensorik notwendig macht.

Anabolika, besonders anabole Steroide, werden häufig zum Doping eingesetzt. Die erwünschte Wirkung der Anabolika beruht auf einer besseren Fettverbrennung bei gleichzeitigem Muskelaufbau. Sie sorgen für eine positive Stickstoffbilanz und damit für eine muskelanabole Stoffwechsellage. Neben den natürlichen Androgenen werden verstärkt synthetische Steroide, die eine dem männlichen Sexualhormon Testosteron ähnliche Wirkung zeigen, zum Doping verwendet.

Durch den umfangreichen Einsatz von Hormonen sowohl im medizinischen Bereich als auch zu illegalen Leistungssteigerungen ist die Umweltbelastung durch hormonell wirksame Substanzen stark gestiegen. Verabreichte Hormone werden teils mit dem Urin wieder ausgeschieden und gelangen so in die Gewässer, da sie durch herkömmliche Kläranlagen nicht aus dem Abwasser gefiltert werden. Da Hormone und hormonell wirksame Substanzen mit hoher Affinität an die entsprechenden Rezeptoren binden und bereits in kleinsten Mengen ihre Wirksamkeit entfalten, stellt die Belastung von Gewässern ein großes Problem für Mensch und Tier dar.

Abgesehen von synthetischen Hormonen, die in die Umwelt gelangen, können auch Naturstoffe hormonelle Wirksamkeit entfalten. Viele pflanzliche Stoffe besitzen hormonelle Wirkung und werden daher auch als Phytohormone bezeichnet. Diesen Stoffen wird oftmals eine positive Wirkung zugeschrieben, weshalb sie auch therapeutisch eingesetzt werden, beispielsweise bei Wechseljahresbeschwerden.

Die klassische analytische Methode zum Nachweis von Steroidhormonen ist die Gaschromatographie, gefolgt von einer Massenspektrometrie (GC/MS). Ein Nachteil dieser Methode besteht darin, dass vom Prinzip her nur bekannte Substanzen nachgewiesen werden können. Dies ist beispielsweise dann problematisch, wenn zum Zwecke des Dopings synthetische Substanzen verwendet werden, die den Dopingfahndern bisher noch unbekannt sind. Diese können mit den herkömmlichen Verfahren kaum erfasst werden.

Für die Identifizierung von neuen, in der Umwelt vorkommenden, hormonell wirksamen Substanzen, die keine Hormone sind, aber im menschlichen oder tierischen Organismus hormonelle Wirkung entfalten können (sogenannte Xenohormone, zu denen auch die oben erwähnten Phytohormone gehören), sind derartige Verfahren ebenfalls nur sehr bedingt geeignet.

Eine deutliche Verbesserung bei diesem Problem stellen biologische Testverfahren dar, die Substanzen über die Bindung an den entsprechenden Rezeptor erkennen. Hier liegen sowohl sehr aufwendige und kostenintensive Säugerzellsysteme, als auch deutlich einfachere und preiswertere Hefesysteme vor. Zwei häufig angewendete Hefesysteme sind der Estrogenrezeptor-α und der Androgenrezeptor Hefe Assay von Sumpter und Mitarbeitern (Routledge and Sumpter, 1996; Sohoni and Sumpter, 1998). Hier führt die Rezeptorbindung zur Erhöhung einer Enzymaktivität, die dann zu einem Farbumschlag des Mediums oder zu Biolumineszenz führen. Die bisherigen Lösungen der oben erwähnten Hefe Assays haben den Nachteil, dass zum Nachweis chemische Reaktionen, wie z.B. Farbreaktionen beim ß-Galaktosidase- oder Phytase-basierten Verfahren, oder physikalische Messgrößen wie die Fluoreszenz von Proteinen, notwendig sind. Außerdem bieten sie keine Möglichkeit einer intrinsischen Signalverstärkung.

Aus US 6,159,705 A ist ein Nachweissystem für das Screening und die Identifizierung pharmazeutisch aktiver Substanzen bekannt, wobei die pharmazeutisch aktive Substanze mit einem zellulären Rezeptor einer Hefezelle interagiert und dessen Aktivität beeinflusst. Der zelluläre Rezeptor ist funktional in einen endogenen Signalübertragungsweg der Hefe integriert, so dass eine Signalveränderung detektierbar ist.

Die in WO 2009/000918 A1 offenbarte Einrichtung betrifft ein intrazelluläres Kommunikationssystem für die Detektion und Verstärkung eines Primärsignals, enthaltend Zellen eines ersten Typs und Zellen eines zweiten Typs, wobei das von einer Zelle des ersten Typs aufgenommene Primärsignal zu der Sekretion eines Signalmoleküls führt, das von den Zellen des zweiten Typs verstärkt wird. WO 2009/000918 A1 offenbart indes keine heterologen Hormonrezeptoren, die durch eine hormonell wirksame Substanz aktivierbar sind und auch kein gerichtetes Zellwandwachstum und/oder die Bildung von Zygoten von haploiden Hefezellen als erfassbare Antwort auf ein Primärsignal.

US 6,555,325 B1 offenbart eine Vorrichtung zur Detektion von Testverbindungen mit einem membranständigen Rezeptor. Die Vorrichtung besteht aus zwei rekombinanten Hefezelltypen, die durch Zugabe einer zu detektierenden Substanz ein detektierbares Signal generieren. Ein extrazelluläres Signalmolekül, welches selbst nicht die Zellmembran passiert, interagiert mit der extrazellulären Domän. Als Antwort wird eine intrazelluläre Signalübertragungskaskade ausgelöst, die die Expression eines Zielgens indirekt induziert.

Aufgabe der Erfindung ist es, ein einfaches Verfahren zum Nachweis und/oder zur Identifizierung von hormonell wirksamen Substanzen zur Verfügung zu stellen, das auch mit sehr einfachen technischen Mitteln verwirklicht werden kann und mit dem ggf. die Nachweisgrenze für die nachzuweisenden hormonell wirksamen Substanzen herabgesetzt wird. Aufgabe der Erfindung ist es weiterhin, ein Verfahren zum Screening von Substanzbibliotheken und Substanzgemischen zur Verfügung zu stellen, mit dessen Hilfe das Vorhandensein von hormonell wirksamen Substanzen schnell und kostengünstig evaluiert werden kann.

Die Aufgabe wird gelöst durch ein Verfahren zum Nachweis und/oder zur Identifizierung von hormonell wirksamen Substanzen unter Nutzung der Pheromonsysteme von Hefen. Das erfindungsgemäße Verfahren umfasst die folgenden Schritte:
a) eine Probe, die mutmaßlich eine hormonell wirksame Substanz umfasst, wird mit Hefekulturmedium bzw. Puffer versetzt,
b) die mit dem Hefekulturmedium bzw. Puffer versetzte Probe wird mit haploiden Hefezellen eines einheitlichen Typs kontaktiert, die die folgenden Merkmale 1 bis 3 umfassen:
   1. die für einen heterologen, nukleären Hormonrezeptor kodierende DNA-Sequenz ist unter die Kontrolle eines konstitutiven Promotors gestellt und wird funktionell exprimiert,
   2. die für ein Hefe-Pheromon kodierende DNA-Sequenz ist unter die Kontrolle eines durch den heterologen, nukleären Hormonrezeptor regulierbaren Promotors gestellt,
   3. ein Gen, das für einen Rezeptor für das Hefe-Pheromon kodiert, wird konstitutiv und funktionell exprimiert,
   wobei die Aktivierung des heterologen, nukleären Hormonrezeptors durch die hormonell wirksame Substanz zu der Expression der für das Hefe-Pheromon kodierenden DNA-Sequenz und zu der Sekretion des Hefe-Pheromons aus der haploiden Hefezelle führt, und
c) die physiologischen und/oder morphologischen Änderungen von haploiden Hefezellen werden erfasst.

Bei Anwesenheit einer hormonell wirksamen Substanz wird der durch die haploiden Hefezellen eines einheitlichen Typs exprimierte heterologe, nukleäre Hormonrezeptor aktiviert. Diese Aktivierung ruft die Expression und Sekretion des Hefe-Pheromons hervor, das unter der Kontrolle eines Hormonrezeptor-regulierbaren Promotors steht.

Haploide Hefezellen, die über einen Rezeptor für das exprimierte und sezernierte Hefe-Pheromon verfügen, reagieren auf die Bindung des Hefe-Pheromons mit physiologischen und/oder morphologischen Änderungen, die anschließend erfasst werden. Die Erfindung macht sich dabei das Pheromonsystem von Hefen zunutze, das bei der Hefepaarung zum Einsatz kommt. Das erfindungsgemäße Verfahren bietet dadurch die Möglichkeit, eine hormonell wirksame Substanz mittels des Hefe-Pheromonsystems und bevorzugt durch einfache, besonders bevorzugt lichtmikroskopische, Verfahren nachzuweisen und/oder zu identifizieren.

Bestandteil der Erfindung sind daher auch zur Durchführung des Verfahrens geeignete haploide Hefezellen eines einheitlichen Typs mit den folgenden Merkmalen:
1. die für einen heterologen, nukleären Hormonrezeptor kodierende DNA-Sequenz ist unter die Kontrolle eines konstitutiven Promotors gestellt und wird funktionell exprimiert.
2. die für ein Hefe-Pheromon kodierende DNA-Sequenz ist unter die Kontrolle eines durch den heterologen, nukleären Hormonrezeptor regulierbaren Promotors gestellt,
wobei die Aktivierung des heterologen, nukleären Hormonrezeptors durch die hormonell wirksame Substanz zu der Expression der für das Hefe-Pheromon kodierenden DNA-Sequenz und zu der Sekretion des Hefe-Pheromons führt,
wobei die haploide Hefezellen eines einheitlichen Typs ein Gen, das für einen Rezeptor für das Hefe-Pheromon kodiert, konstitutiv und funktionell exprimieren.

In einer Ausgestaltungsform der Erfindung wird in dem erfindungsgemäßen Verfahren ein einheitlicher Typ haploider Hefezellen eingesetzt, die einen heterologen Hormonrezeptor, ein unter Kontrolle des heterologen Hormonrezeptors stehendes Gen für ein Hefe-Pheromon und einen Rezeptor für dieses Hefe-Pheromon besitzen, d.h. die alle Merkmale 1 bis 3 umfassen. Dies ermöglicht vorteilhaft die einfachere Handhabung der verwendeten Hefezellen.

Die hormonell wirksamen Substanzen befinden sich vorzugsweise in komplexen Gemischen wie Urinproben, Blutproben, Plasmaproben, Serumproben, Pflanzenextrakten oder Gewebeextrakten. Es kann sich aber auch um synthetische oder natürliche Substanzbibliotheken handeln, in denen Verbindungen in reiner Form oder als Kombinationspräparate aus Einzelsubstanzen vorliegen. Für die Kontaktierung mit den Hefezellen werden Proben dieser Substanzen oder Substanzgemische mit Hefekulturmedium oder Puffer versetzt. Bevorzugt beträgt dabei der Anteil des Hefekulturmediums oder des Puffers von mehr als 50 % bis 99,9 %, bevorzugt von mehr als 20 % bis 99,9% der Mischung. Die Proben können auch direkt in dem gewünschten Verhältnis mit den in Hefekulturmedium befindlichen haploiden Hefezellen vermischt werden. Der Anteil der Probe am Gesamtvolumen beträgt bei der Kontaktierung der Hefezellen mit der Probe vorzugsweise 0,01 % bis 50 %, bevorzugt 0,01 % bis 20 %.

Erfindungsgemäße Hefekulturmedien sind dem Fachmann geläufige Hefekulturmedien wie beispielsweise YPD (Yeast Peptone Dextrose), YPDA (Yeast Peptone Dextrose Adenine), Mangelmedien wie SD (synthetic defined), MM (minimal medium), SMM (supplemented medium) etc. Mangelmedien sind bevorzugt mit ausgewählten Aminosäuren und anderen essentiellen Substanzen versetzt, um die Heranzucht von haploiden Hefezellen mit bestimmten Auxotrophien oder von haploiden Hefezellen, die über spezifische Wachstumsmarker verfügen, zu ermöglichen. Die Zusammensetzung entsprechender Medien ist dem Fachmann geläufig und kann einschlägiger Literatur, wie z.B. Kaiser *et al.* (1994), oder Internet-Seiten verschiedenster mit Hefe arbeitender Labore entnommen werden.

Erfindungsgemäße Puffer sind alle Puffer, die die Überlebensfähigkeit und Responsivität der haploiden Zellen nicht beeinträchtigen. Dazu gehören beispielsweise PBS (Phosphatgepufferte Saline), TBS (Tris-gepufferte Saline) und können einschlägiger Literatur, wie z.B. Kaiser *et al.* (1994), entnommen werden.

Durch die Bindung des Hefe-Pheromons an den Pheromonrezeptor wird ein umfangreiches Paarungsprogramm ("mating response pathway") in den responsiven Hefezellen aktiviert (Leberer et al., 1997). Paarungsspezifische Gene werden induziert und der Zellzyklus arretiert. Anschließend erfolgen umfangreiche morphologische Veränderungen, wie ein gerichtetes Zellwandwachstum (Paarungsprojektion) der Zellen zur Quelle des Hefe-Pheromons, in der Regel zum Paarungspartner (Jackson et al., 1991; Segall, 1993). Dieses "Ausstrecken" der Hefezellen wird als "Shmoo" bezeichnet (Mackay und Manney, 1974).

Innerhalb von 1-2 h nach der Aktivierung des Pheromonrezeptors durch die Bindung des Hefe-Pheromons sind die morphologischen Änderungen gut sichtbar. Neben dem gerichteten Wachstum zum Paarungspartner, der durch das entsprechende Hefe-Pheromon induziert wird, beobachtet man in Zellpopulationen unterschiedlichen Paarungstyps die Fusion der Zellen und die Bildung charakteristisch geformter Zygoten (Tkacz and MacKay, 1979). Der gesamte Vorgang der Paarung bis zur diploiden Zygote lässt sich lichtmikroskopisch sehr einfach und effektiv verfolgen. Zunächst werden auf der Zelloberfläche der Zellen unterschiedlichen Paarungstyps Agglutinine exponiert, wodurch es zu einer Bindung der Zellen aneinander bzw. zu einer Verklumpung kommt. Die Zellen adhärieren, es kommt zur Fusion der Zellwände, der Zellmembranen, zur Cytogamie und Karyogamie (Marsh and Rose, 1997).

Diese durch das Einwirken der Hefe-Pheromone hervorgerufenen morphologischen Änderungen können vorteilhaft mit einem Lichtmikroskop beobachtet werden. Dazu ist eine mindestens 100fache, bevorzugt 200 bis 1.000fache Vergrößerung, geeignet.

Die Detektion ist auch über die Trübung der Lösung, in der sich die erfindungsgemäßen haploiden Hefezellen befinden, messbar und kann mittels eines Nephelometers erfasst werden. Diese Messungen sind vorteilhaft mit geringem technischem Aufwand durchführbar.

Eine weitere morphologische Änderung, die mit der Hefe-Paarung einhergeht und die zur Detektion der Androgenwirkung genutzt werden kann, ist der durch die bei der Paarung sezernierten Pheromone bewirkte Zellzyklusarrest. Durch das Pheromon des einen Paarungstyps wird in Zellen des anderen Paarungstyps die Mitose als Vorbereitung auf die Paarung und die darauffolgende Meiose blockiert.

Man kann sich diesen Effekt zunutze machen, um die Hormonwirkung einer Substanz im erfindungsgemäßen Verfahren zu detektieren. Inkubiert man ein Aliquot von haploiden Hefezellen, die die Merkmale 1 bis 3 aufweisen, mit einer hormonell wirksamen Substanz und bringt sie dann punktuell auf eine Agar-Platte auf, die flächig mit stark verdünnten haploiden Hefezellen, beimpft wurde, dann hören letztere Zellen, bewirkt durch das abgegebene Hefepheromon der mit der zu testenden Substanz inkubierten Hefezellen, auf zu wachsen. Im übrigen Bereich der Agarplatte können die Hefezellen jedoch ungehindert wachsen. Dadurch bildet sich dort, wo die mit der zu testenden Substanz inkubierten Hefezellen punktuell aufgebracht wurden, eine mit bloßem Auge sichtbare Zone ohne Hefezellen (Hemmhof).

Alternativ oder auch zusätzlich zu der Erfassung der morphologischen Änderungen werden in den haploiden Hefezellen physiologische Änderungen erfasst. Dazu ist in den haploiden Hefezellen ein spezifisches Gen, das beispielsweise für ein Markerprotein, wie z.B. GFP (grün fluoreszierendes Protein) kodiert, unter die Kontrolle eines Promotors gestellt, der durch das Hefe-Pheromon reguliert wird, welches durch die haploiden Hefezellen eines einheitlichen Typs mit den Merkmalen 1 bis 3 sezerniert wird. Das heißt, dass die haploiden Hefezellen gentechnisch so verändert sind, dass sie auf die Aktivierung des Hefe-Pheromon-Rezeptors durch physiologische Änderungen reagieren, welche physikalisch oder chemisch nachweisbar sind. Erreicht das durch die Bindung der hormonell wirksamen Substanz an den heterologen Hormonrezeptor exprimierte und sezernierte Hefe-Pheromon die umliegenden Zellen, die einen Rezeptor für das Hefe-Pheromon besitzen, wird in diesen Zellen als eine physiologische Änderung die Expression des spezifischen Gens und die Produktion des Markerproteins oder Zielproteins durch den Hefe-Pheromon-regulierten Promotor hervorgerufen.

Bei dem Markerprotein handelt es sich beispielsweise um ß-Galaktosidase, EGFP oder Phytase. Durch die Hefe-Pheromon-kontrollierte Expression des spezifischen Gens wird ein nachweisbares Signal wie z.B. Farbreaktionen oder Fluoreszenz erzeugt. Dadurch wird vorteilhaft die erfindungsgemäße Detektion des Hormons auch durch die Erfassung anderer Messparameter als der morphologischen Änderungen der Hefezellen ermöglicht und erleichtert, beispielsweise durch Fluoreszenzmessung oder durch enzymatische Farbreaktionen.

Markerproteine im Sinne der Erfindung sind daher alle Proteine, deren Vorhandensein bzw. Aktivität zu einer physikalisch und/oder chemisch messbaren Änderung führt. Diese physikalisch und/oder chemisch messbare Änderung wird durch ein geeignetes Nachweissystem einfach und/oder schnell detektiert. Bevorzugt werden solche Markerproteine verwendet, die nachgewiesen werden können, ohne die Integrität bzw. Vitalität der Zellen zu beeinträchtigen, wie z. B. Enzyme, die in Anwesenheit eines Substrats eine Farbreaktion katalysieren, wie ß-Galaktosidase oder Phytase. Weiter bevorzugt werden als Markerproteine Luciferasen, die in Anwesenheit eines geeigneten Substrats Licht aussenden. Besonders bevorzugt werden als Markerproteine Proteine, die durch Anregung mit Licht einer bestimmten Wellenlänge fluoreszieren. Weiterhin umfasst die Erfindung Proteasen, die fluoreszierende Proteine abbauen, als Markerproteine. Exprimiert die haploide Hefezelle gleichzeitig konstitutiv ein fluoreszierendes Protein, wird auf die Detektion des Hormons hin die Abnahme der Fluoreszenz der Zellen messbar. Bevorzugt verwendet werden Proteasen, die außer dem fluoreszierenden Protein keine weiteren Ziele in der Zelle angreifen, um die Vitalität der Zelle nicht zu beeinträchtigen. Besonders bevorzugt verwendet wird die TEV-Protease. Die entsprechenden fluoreszierenden Proteine müssen ggf. mittels rekombinanter DNA-Techniken so verändert werden, dass sie die Erkennungssequenz für die entsprechende Protease enthalten und so abbaubar sind.

Das Markerprotein ist bevorzugt ein fluoreszierendes Protein, wobei die Expression des entsprechenden Markerproteins nach Sezernierung des Hefe-Pheromons durch die haploiden Hefezellen variiert, was zu einer Zu- oder Abnahme der Fluoreszenz der haploiden Hefezelle führt. Vorzugsweise verwendet werden die fluoreszierenden Proteine GFP, YFP, CFP, BFP, RFP, DsRed, PhiYFP, JRed, emGFP ("Emerald Green"), Azami-Green, Zs-Green oder AmCyan 1. Bevorzugt verwendet werden Proteine, die so verändert wurden, dass sie besonders stark fluoreszieren wie eGFP, GFPuv, eYFP, TagCFP, TagGFP, TagYFP, TagRFP und TagFP365. Weiterhin werden bevorzugt solche fluoreszierenden Proteine verwendet, deren Aminosäuresequenz dahingehend verändert ist, dass sie nach einer möglichst kurzen Reifungszeit fluoreszieren. Bevorzugt verwendet werden dabei TurboGFP, TurboYFP, TurboRFP, TurboFP602, TurboFP635, und dsRed-Express.

Besonders bevorzugt kodiert das durch das Hefe-Pheromon regulierte spezifische Gen für ein grün, (z. B. GFP, GFPuv), gelb (z. B. YFP), blau (z. B. BFP), cyan (z. B. CFP) oder rot (z. B. dsRed) fluoreszierendes Protein als Markerprotein.

Mittels eines geeigneten Nachweissystems für das Markerprotein wird die Bildung des Markerproteins sensortechnisch erfasst. Bevorzugt wird die Bildung des Markerproteins durch ein Fluorimeter, ein Spektrometer, ein Mikroskop, einen Plattenreader, oder durch Flow-Cytometrie erfasst.

Die erfindungsgemäßen Hormonrezeptoren sind heterologe Hormonrezeptoren, da sie nicht authentisch in den verwendeten haploiden Hefezellen vorhanden sind, sondern anderen Organismen entstammen. Sie werden durch die haploiden Hefezellen exprimiert, indem eine für den heterologen Hormonrezeptor kodierende DNA-Sequenz in die haploiden Hefezellen eingebracht wird. Die heterologen Hormonrezeptoren sind vorzugsweise intrazelluläre, bevorzugt cytoplasmatische bzw. nukleäre Hormonrezeptoren. Niedermolekulare Hormone, wie beispielsweise Steroidhormone, die durch Zell- und Kernmembranen diffundieren können, binden in der Regel an solche intrazellulären Rezeptoren. Die Rezeptoren liegen dabei oft nukleär, d. h. innerhalb des Zellkerns, vor. Solche nukleär vorliegenden Rezeptoren werden daher auch als nukleäre Rezeptoren oder Kernrezeptoren bezeichnet. Sie bestehen in der Regel aus einer Hormon-bindenden Domäne und einer DNA-bindenden Domäne. Durch die DNA-Bindung nehmen sie an der Regulation bestimmter Zielgene teil. Bei diesen Kernrezeptoren handelt es sich meist um Proteine mit einer Zinkfingerdomäne, die für die DNA-Bindung verantwortlich ist. Beispiele für solche Kernrezeptoren sind die Rezeptoren für das Schilddrüsenhormon (Triiodthyronin), für die Steroid-Hormone, für Vitamin D3, für Retinsäure und für Gallensäuren.

Diese intrazellulären bzw. nukleären Hormonrezeptoren ermöglichen vorteilhaft die Detektion von Hormonen, ohne dass sichergestellt werden muss, dass die verwendeten haploiden Hefezellen auch über geeignete second messenger-Systeme für die Weiterleitung des Hormonsignals an den Zellkern verfügen. Intrazelluläre und nukleäre Hormonrezeptoren binden vorteilhaft direkt an die DNA im Zellkern und regulieren so die Genexpression.

Hormonrezeptoren sind hochspezifisch und zudem hochaffin, da Hormone im Körper üblicherweise nur in kleinsten Mengen vorhanden sind. Dadurch ermöglichen sie vorteilhaft die hochsensitive Erfassung kleinster Mengen hormonell wirksamer Substanzen. Bevorzugte Hormonrezeptoren sind in Tab. 1 aufgelistet. Vorzugsweise werden Rezeptoren für Steroidhormone verwendet, besonders bevorzugt sind die erfindungsgemäßen Hormonrezeptoren ausgewählt aus Estrogenrezeptoren und Androgenrezeptoren.

**Tab. 1: Bevorzugte erfindungsgemäße Hormonrezeptoren**

| Subfamilien/ Gruppen | Gen | Trivialnamen | Alternative Namen | Genbank Accession number |
|---|---|---|---|---|
| 1A | NR1A1 | TRa, c-erbA-1, THRA | Thyroid hormone receptor-a | M24748 |
| | NR1A2 | TRb, c-erbA-2, THRB | Thyroid hormone receptor-b | X04707 |
| 1B | NR1B1 | RARa | Retinoic Acid receptor-a | X06538 |
| | NR1B2 | RARb, HAP | Retinoic Acid receptor-b | Y00291 |
| | NR1B3 | RARg, RARD | Retinoic Acid receptor-g | M57707 |
| | NR1B4 | RAR | Retinoic Acid Receptor | AF378827 |
| 1C | NR1C1 | PPARa | Peroxisom Proliferator Aktivierter Rezeptor-a | L02932 |
| | NR1C2 | PPARb, NUC1, PPARd, FAAR | Peroxisom Proliferator Aktivierter Rezeptor-b | L07592 |
| | NR1C3 | PPARg | Peroxisom Proliferator Aktivierter Rezeptor-g | L40904 |
| 1D | NR1D1 | REVERBa, EAR1, EAR1A | | M24898 |
| | NR1D2 | REVERBb, EAR1b, BD73, RVR, HZF2 | | L31785 |
| | NR1D3 | E75 | | X51548 |
| 1E | NR1E1 | E78, DR-78 | | U01087 |
| 1F | NR1F1 | RORa, RZRa | retinoid-related orphan receptor-a | U04897 |
| | NR1F2 | RORb, RZRb | retinoid-related orphan receptor-b | Y08639 |
| | NR1F3 | RORg, TOR | retinoid-related orphan receptor-g | U16997 |
| | NR1F4 | HR3, DHR3, MHR3, GHR 3 | | M90806 |
| | | CHR3, CHR3 | | U13075 |
| 1G | NR1G1 | CNR14 | | U13074 |
| 1H | NR1H1 | ECR | | M74078 |
| | NR1H2 | UR, OR-1, NER1, RIP15, LXRb | Liver X Receptor-b | U07132 |
| | NR1H3 | RLD1, LXR, LXRa | Liver X Receptor-a | U22662 |
| | NR1H4 | FXR, RIP14, HRR1 | Farnesoid X Rezeptor | U09416 |
| | NR1H5 | FXRB | Farnesoid X Rezeptor-b | AY09458 6 |
| 1I | NR1I1 | VDR | Vitamin-D Rezeptor | J03258 |
| | NR1I2 | ONR1, PXR, SXR, BXR | Pregnan-X-Rezeptor | X75163 |
| | NR1I3 | MB67, CAR1, CARa | | Z30425 |
| | NR1I4 | CAR2, CARb | | AF009327 |
| 1J | NR1J1 | DHR96 | | U36792 |
| 1K | NR1K1 | NHR1 | | U19360 |
| 2A | NR2A1 | HNF4 | Hepatic nuclear factor 4 | X76930 |
| | NR2A2 | HNF4G | Hepatic nuclear factor 4G | Z49826 |
| | NR2A3 | HNF4B | Hepatic nuclear factor 4B | Z49827 |
| | NR2A4 | DHNF4, HNF4D | | U70874 |
| 2B | NR2B1 | RXRA | Retinoic X-receptor-a | X52773 |
| | NR2B2 | RXRB, H-2RIIBP, RCoR-1 | Retinoic X-receptor-b | M84820 |
| | NR2B3 | RXRG | Retinoic X-receptor-g | X66225 |
| | NR2B4 | USP, Ultraspiracle, 2C1, CF1, RXR1, RXR 2 | | X52591 |
| 2C | NR2C1 | TR2, TR2-11 | | M29960 |
| | NR2C2 | TR4, TAK1 | | L27586 |
| | NR2C3 | TR2-4 | | AF378828 |
| 2D | NR2D1 | DHR78 | | U36791 |
| 2E | NR2E1 | TLL, TLX, XTLL | | S72373 |
| | NR2E2 | TLL, Tailless | | M34639 |
| | NR2E3 | PNR | | AF121129 |
| | NR2E4 | dissatisfaction | | 096680 |
| | NR2E5 | fax-1 | | Q9U410 |
| 2F | NR2F1 | COUP-TFI, COUPTFA, EAR3, SVP44 | Chicken ovalbumin upstream promotertranscription factor-I | X12795 |
| | NR2F2 | COUP-TFII, COUPTFB, ARP!, SVP40 | Chicken ovalbumin upstream promotertranscription factor-II | M64497 |
| | NR2F3 | SVP, COUP-TF | | M28863 |
| | NR2F4 | COUP-TFIII, COUPTFG | | X63092 |
| | NR2F5 | SVP46 | | X70300 |
| | NR2F6 | EAR2 | | X12794 |
| | NR2F7 | AmNR7 | | AF323687 |
| 2G | NR2G1 | HNF, RXR | | AJ517420 |
| 2H | NR2H1 | AmNR4, AmNR8 | | AF323683 |
| 3A | NR3A1 | ERa | Estrogen receptor-a | X03635 |
| | NR3A2 | ERb | Estrogen receptor-b | U57439 |
| 3B | NR3B1 | ERR1, ERRa | Estrogen related receptor-a | X51416 |
| | NR3B2 | ERR2, ERRb | Estrogen related receptor-b | X51417 |
| | NR3B3 | ERR3, ERRg | Estrogen related receptor-g | AF094318 |
| | NR3B4 | Drosophila ERR | | AE003556 |
| 3C | NR3C1 | GR | Glucocorticoid receptor | X03225 |
| | NR3C2 | MR | Mineralocorticoid receptor | M16801 |
| | NR3C3 | PR | Progesteron receptor | M15716 |
| | NR3C4 | AR | Androgen receptor | M20132 |
| 4A | NR4A1 | NGFIB, TR3, N10, NUR77, NAK1 | | L13740 |
| | NR4A2 | NURR1, NOT, RNR1, HZF-3, TINOR | | X75918 |
| | NR4A3 | NOR1, MINOR | | D38530 |
| | NR4A4 | DHR38, NGFIB | | U36762 |
| | | CNR8, C48D5 | | U13076 |
| 5A | NR5A1 | SF1, ELP, FTZ-F1, AD4BP | | D88155 |
| | NR5A2 | LRH1, xFF1rA, xFF1rB, FFLR, PHR, FTF | | U93553 |
| | NR5A3 | FTZ-F1 | | M63711 |
| | NR5A4 | FF1b | | Q9IAI9 |
| 5B | NR5B1 | DHR39, FTZF1B | | L06423 |
| 6A | NR6A1 | GCNF1, RTR | | U14666 |
| | NR6A2 | HR4, THR4, GRF | | AL035245 |
| 0A | NR0A1 | KNI, Knirps | | X13331 |
| | NR0A2 | KNRL, Knirps related | | X14153 |
| | NR0A3 | EGON, Embryonic gonad, EAGLE | | X16631 |
| | NR0A4 | ODR7 | | U16708 |
| | NR0A5 | Trithorax | | M31617 |
| 0B | NR0B1 | DAX1, AHCH | | S74720 |
| | NR0B2 | SHP | | L76571 |

Ein besonders bevorzugter Hormonrezeptor der Erfindung ist der humane Androgenrezeptor (hAR), an den androgen-wirksame Hormone wie Testosteron oder dessen Metabolit Dihydrotestosteron binden. Er gehört zur Familie der nukleären Rezeptoren. Es existieren zwei Isoformen des Rezeptors, Isoform 1 (87 kDa) und Isoform 2 (110 kDa) (Wilson und McPhaul, 1994), welche durch das h*AR*-Gen codiert werden. Die Isoformen unterscheiden sich darin, dass der Isoform 1 23 Aminosäuren am N-Terminus fehlen. Strukturell gesehen besteht der Androgenrezeptor aus vier funktionellen Domänen. Am N-Terminus weist er eine N-terminale Domäne auf, durch die vor allem die Aktivierung des Rezeptors reguliert wird. Dabei wird sowohl die Aktivierung durch androgen-wirksame Hormone, wie Testosteron oder Dihydrotestosteron (Roy et al., 1999), als auch die konstitutive Aktivierung ohne Ligand gesteuert (Jenster et al., 1995). Zudem spielt diese Domäne bei der Dimerisierung des Rezeptors eine Rolle (Langley et al., 1995; Doesburg et al., 1997). Auf die N-terminale Domäne folgt die stark konservierte DNA-Bindungsdomäne, mit welcher der Rezeptor an die DNA bindet. Zwischen DNA-Bindungs- und Liganden-Bindungsdomäne befindet sich die so genannte *hinge*-Region. Diese ist ein flexibler, kaum konservierter Bereich des Rezeptors. Diese Region koordiniert die Lokalisation des Rezeptors im Zellkern und enthält einen Teil der nukleären Signalsequenz. Am C-Terminus des Rezeptorproteins befindet sich die Liganden-Bindungsdomäne. Dieser Bereich besitzt des Weiteren eine Aktivierungsfunktion, welche für Agonisten von Bedeutung ist. In dieser Region können auch Coaktivatoren binden (Dubbink et al., 2004).

Bei der Funktion des Androgenrezeptors kann zwischen einer genomischen und einer nichtgenomischen Funktion unterschieden werden. Die Hauptfunktion des Rezeptors ist die genomische. Die Anlagerung des Liganden bewirkt unter anderem die Dimerisierung des Rezeptorproteins und den Transport des Rezeptor-Liganden-Komplexes in den Zellkern. Dort bindet das Konstrukt an einer spezifischen DNA-Sequenz, dem Hormon-responsiven Element. Somit ist der Androgenrezeptor ein Transkriptionsfaktor, welcher die Expression von Genen steuert (Mooradian et al., 1987). Beispiele für die nicht-genomische Funktion sind die Aktivierung der MAPK-Signalkaskade und die Steuerung der intrazellulären Calciumkonzentration.

Hormonrezeptoren im Sinne der Erfindung sind nicht nur die natürlichen Proteine, sondern auch Derivate davon, beispielsweise chimäre Proteine, die durch Fusion des Hormonrezeptors mit anderen Proteinen oder Proteindomänen oder die Verknüpfung verschiedener Domänen verschiedener Hormonrezeptoren zu neuen Proteinen erzeugt werden, z.B. durch molekularbiologische Techniken.

Die Verwendung von Hormonrezeptoren ermöglicht vorteilhaft auch die Detektion von unbekannten hormonell wirksamen Substanzen, d. h. von Substanzen, die nicht bekannt sind, oder von bekannten Substanzen, deren hormonelle Wirksamkeit nicht bekannt ist. Das Verfahren eignet sich daher sowohl für den Nachweis von hormonell wirksamen Substanzen in Proben als auch zum einfachen und kostengünstigen Screening von Substanzen zur Einschätzung ihrer hormonellen Wirksamkeit.

Zu den erfindungsgemäßen hormonell wirksamen Substanzen gehören Hormone. Hormone sind biochemisch uneinheitliche Substanzen, die innerhalb eines Organismus Signale und Botschaften an Zellen, Zellgruppen, Gewebe oder Organe, die vom Bildungsort der Hormone entfernt liegen, übermitteln und so eine bestimmte physiologische Wirkung auf deren Funktion ausüben. Die erfindungsgemäßen Hormonrezeptoren binden in erster Linie an Steroidhormone, aber auch an andere niedermolekulare Hormone, die ausreichend lipophil sind, um durch die Zell- bzw. ggf. die Kernmembran zu diffundieren. Erfindungsgemäße Hormone sind Nicht-Proteohormone, beispielsweise Steroide (Steroid-Hormone), biogene Amine (L-Thyroxin, Melatonin, Catecholamin) und Fettsäure-Derivate (Prostaglandine und andere Eicosanoide).

Hormone, die durch das erfindungsgemäße Verfahren nachgewiesen werden können, sind beispielsweise Steroidhormone wie Estrogene (z.B. Estradiol, Estron und Estriol), Androgene (z.B. Androsteron, Testosteron, Dihydrotestosteron), Gestagene (z.B. Progesteron), Glucocorticoide (z.B. Cortisol, Corticosteron, Cortison), oder Mineralocorticoide (z.B. Aldosteron), und auch Thyrosinderivate wie Adrenalin, Melatonin, Levothyroxin (Tetraiodthyronin) und Liothyronin (Triiodthyronin) oder Vitamine wie Cholecalciferol (kurz Calciol oder auchVitamin D3) oder Retinol (Vitamin A).

Hormone im Sinne der Erfindung sind dabei sowohl die natürlichen Hormone als auch chemisch synthetisierte Verbindungen, die mit den natürlichen Hormonen strukturidentisch sind. Bevorzugte Hormone der Erfindung sind Wirbeltierhormone, besonders Säugerhormone.

Neben natürlichen Hormonen sind hormonell wirksame Substanzen im Sinne der Erfindung auch hormonähnliche Substanzen, die aufgrund ihrer Struktur in der Lage sind, die gleichen oder ähnliche Wirkungen wie natürliche Hormone auszuüben. Dazu gehören Substanzen, die als Xenohormone bezeichnet werden, wie z.B. Phytoestrogene. Bei diesen handelt es sich im Allgemeinen um nichtsteroide Substanzen, die dennoch den Hormonrezeptor aktivieren können. Zu den hormonell wirksamen Substanzen gehören aber auch synthetische Strukturanaloga von natürlichen Hormonen, die an dieselben Rezeptoren binden können wie natürliche Hormone. Dazu gehören beispielsweise sogenannte Designer-Steroide wie Norbolethon, Nandrolon, Stanozolol, Desoxymethyltestosteron (Madol), Tetrahydrogestrinon, Methandrostenolon (Metandienon), aber auch Estrogene wie Ethinylestradiol, Mestranol, Stilbestrol, desweiteren Antihormone wie die Antiandrogene wie Cyproteroneacetat, Flutamid oder Bicalutamid, Antiestrogene wie Faslodex, sowie selektive Estrogenrezeptormodulatoren wie Raloxifen, Tamoxifen, Toremifen, Bazedoxifen, Lasofoxifen und selektive Androgenrezeptormodulatoren wie Andarine.

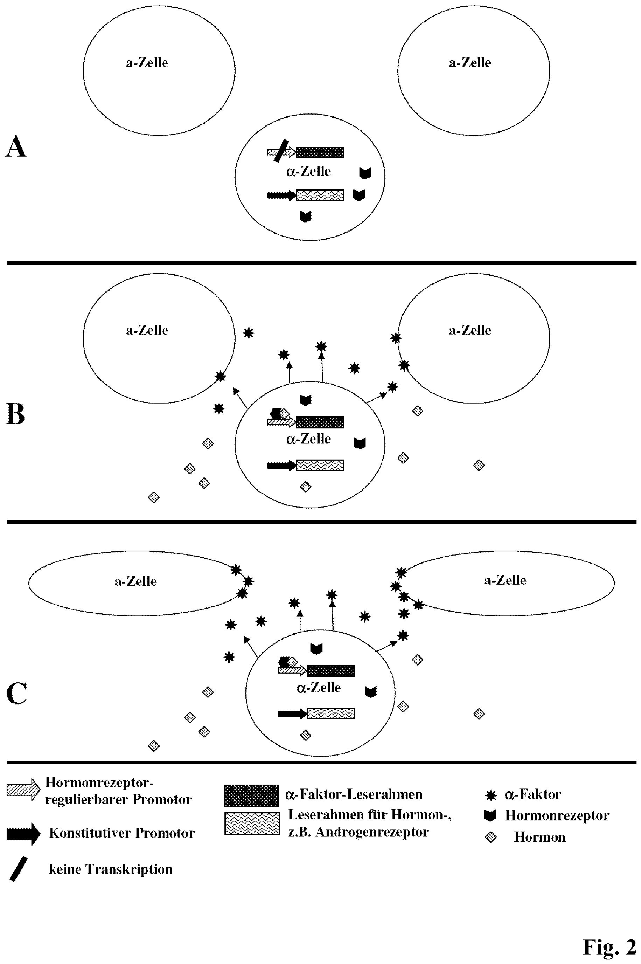

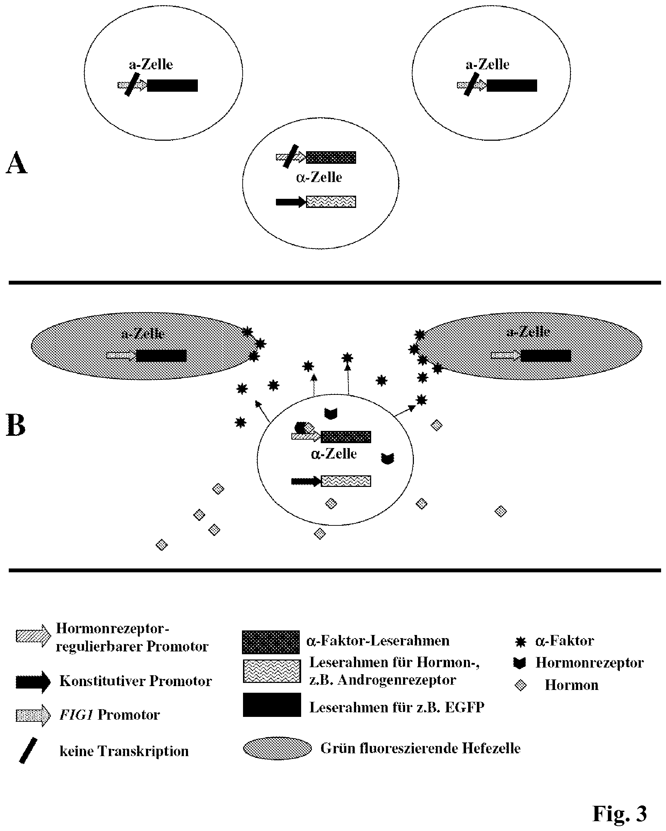

In den erfindungsgemäßen haploiden Hefezellen wird das Gen für den entsprechenden Hormonrezeptor, z.B. den Androgenrezeptor oder den Estrogenrezeptor, vorteilhafterweise unter die Kontrolle eines konstitutiven Promotors gestellt, um die Expression des Hormonrezeptors in der Zelle sicher zu stellen (Fig. 2; Fig. 3).

Als Promotor im Sinne der Erfindung wird eine DNA-Sequenz bezeichnet, die die Expression eines Gens reguliert. In der Regel befinden sich diese regulierenden DNA-Bereiche auf der 5'-Seite des Transkriptionsstartpunkts des betreffenden Gens. Solche regulierenden Bereiche können nahe am Transkriptionsstartpunkt, aber auch weiter als 1000 bp entfernt von der kodierenden Sequenz liegen. Sie können auch auf der 3'-Seite der kodierenden Sequenz des betreffenden Gens oder innerhalb der transkribierten Sequenz des betreffenden Gens liegen.

Durch heterologe Hormonrezeptoren regulierbare Promotoren im Sinne der Erfindung sind bevorzugt diejenigen Bereiche der genomischen DNA, die spezifisch für die Regulation der Expression eines Gens verantwortlich sind, indem sie auf die Anwesenheit von hormonell wirksamen Substanzen reagieren und abhängig von diesen Signalen die Expression des unter ihrer Kontrolle liegenden Gens aktivieren oder reprimieren. Setzt man derartige Promotoren an die 5'-Seite des Transkriptionsstartpunkts eines beliebigen Gens, bevorzugt eines Hefe-Pheromon-Gens, regulieren sie die Aktivität dieses Gens in Abhängigkeit von den oben genannten Hormonrezeptoren.

Konstitutiver Promotor im Sinne der Erfindung bedeutet, dass die unter Kontrolle eines solchen Promotors stehenden Leserahmen oder Gene unter den Bedingungen des erfindungsgemäßen Verfahrens dauerhaft exprimiert werden. Bevorzugt werden solche Promotoren verwendet, die unabhängig von Zelltyp, Zellstadium und Wachstumsbedingungen dauerhaft exprimiert werden.

Die Expression von heterologen Hormonrezeptoren in Hefezellen ist aus dem Stand der Technik bekannt. So können in Hefen zum Beispiel Sensorsysteme für menschliche Hormone wie Androgene (Bovee et al.; 2008) exprimiert werden. Bevorzugte Promotoren für die konstitutive Expression eines Zielgens in Hefezellen sind beispielsweise die *ADH1*-, *GPD*-, *TEF2-* und *CYC1*-Promotoren (Mumberg et al., 1995). Weitere bevorzugte Promotoren sind in Tabelle 2 angegeben. Weitere konstitutive Promotoren sind in einschlägigen Datenbanken, wie z.B. der Saccharomyces Genome Database (SGD, Department of Genetics at the School of Medicine, Stanford University, USA) auf der Basis der ermittelten Expressionsprofile zu finden.

**Tab. 2: Ausgewählte konstitutive Promotoren in Saccharomyces cerevisiae.**

| **Promoter** | **Quellen** |
|---|---|
| *PGK1* | Protchenko et al., 2008 |
| | Sekler et al., 1995 |
| *PGK1, SPT15* | McNabb et al., 2005 |
| *PMA1* | Perez-Castineira et al., 2002 |
| | Opekarova et al., 1998 |
| *SUF14* | Fairman et al., 1999 |
| | Hill et al., 1986 |
| *GAPFL* | Hottiger et al., 1994 |
| | Janes et al., 1990 |
| *TPI1* | Chow et al., 1994 |
| *TDH3* | Chernaik und Huang, 1991 |
| | Ecker et al., 1986 |

Promotoren im Sinne der Erfindung sind auch zu den entsprechenden Hefe-Promotoren homologe DNA-Abschnitte, die eine Sequenzidentität von vorzugsweise mehr als 50%, bevorzugt mehr als 80%, aufweisen. Diese Abschnitte können beispielsweise aus homologen genomischen Bereichen von anderen Organismen, bevorzugt anderen Hefestämmen, stammen. Sie können jedoch auch synthetisch hergestellte DNA-Sequenzen sein, deren Sequenz eine Identität von vorzugsweise mehr als 50%, bevorzugt mehr als 80%, mit dem entsprechenden *Saccharomyces cerevisiae* Promotor aufweist.

Promotoren können auch synthetische DNA-Sequenzen sein, die aus einem Teilbereich eines der oben genannten Hefe-Promotoren oder regulativen DNA-Abschnitten aus anderen Organismen sowie einem bekannten Basalpromotor aus *Saccharomyces cerevisiae* zusammengesetzt sind. Der Basalpromotor stellt die für die Anbindung der Transkriptionsmaschinerie notwendigen DNA-Sequenzen zur Verfügung, während die regulativen DNA-Abschnitte spezifisch auf regulierende Signale reagieren. Ein solcher Basalpromotor ist bevorzugt der Basalpromotor des *Cytochrom* c-Gens aus *Saccharomyces cerevisiae*, der in einem 300 bp Fragment 5'seitig des Startcodons des *Cytochrom* c-Gens enthalten ist (Chen et al., 1994).

Promotoren aus synthetischen DNA-Sequenzen können auch mehrfache Abschnitte einer identischen DNA-Sequenz enthalten. Diese Vervielfachung eines regulatorischen DNA-Abschnitts erlaubt vorteilhaft eine Erhöhung der Sensitivität des Promotors gegenüber den regulierenden Faktoren, wie Transkriptionsfaktoren oder Hormonrezeptoren.

Erfindungsgemäß werden die haploiden Zellen außerdem genetisch so verändert, dass ein Gen, das für ein Paarungstyp-spezifisches Hefe-Pheromon kodiert, unter die Kontrolle eines Promotors gestellt ist, der durch den heterologen Hormonrezeptor reguliert wird (Fig. 2; Fig. 3). Der Begriff Promotor ist hier wie oben definiert zu verstehen.

Durch Hormonrezeptoren regulierte Promotoren sind gekennzeichnet durch Bindestellen für die betreffenden Hormonrezeptoren, sogenannte hormone response elements (*HREs*), wie beispielsweise androgen response elements (*ARE*) oder estrogen response elements (*ERE*). Besonders bevorzugt ist das von Sohoni & Sumpter (1998) beschriebene Derivat des *PGK-*Promotors mit 3 AREs (SEQ ID No. 1).

Dargestellt ist hier die Sequenz des Derivates des *PGK*-Promotors: Sequenz des *PGK-*Promotors (kursiv) sowie der drei *androgen response elements* (*AREs*) (fett gedruckt) im p426PGK-Vektor.

Bevorzugt wird der *PGK*-Promotor auch dazu genutzt, um durch die Kombination mit anderen hormone reponse elements neue Promotoren zu erzeugen, die durch andere Hormonrezeptoren als den Androgen-Rezeptor reguliert werden Dazu können z.B. die im *PGK*-Promotorderivat (SEQ ID No. 1) enthaltenen AREs durch entsprechende andere responsive Elemente ausgetauscht werden.

Die Aktivierung des durch den heterologen Hormonrezeptor regulierten Promotors führt zu der Expression und Sekretion des Hefe-Pheromons. Hefe-Pheromone spielen bei der Hefe-Paarung eine wichtige Rolle. Hefezellen können grundsätzlichen sowohl im diploiden Zustand als auch im haploiden Zustand vorliegen. Zwei haploide Hefezellen können in einem Vorgang, der als Hefe-Paarung bezeichnet wird, zu einer einzigen diploiden Hefezelle verschmelzen. Bei haploiden Hefezellen unterscheidet man dabei zwei so genannte Paarungstypen. Nur Hefezellen mit unterschiedlichem Paarungstyp können miteinander paaren. Beispielsweise sind dies bei der Bäckerhefe *Saccharomyces cerevisiae* die Paarungstypen α und a, bei der Spalthefe *Schizosaccharomyces pombe* die Paarungstypen Plus und Minus.

Die erfindungsgemäßen haploiden Hefezellen sind *Saccharomyces cerevisiae* oder *Schizosaccharomyces pombe* Zellen. Bevorzugt sind die haploiden Hefezellen dabei *Saccharomyces cerevisiae*-Zellen des α-Paarungstyps oder *Saccharomyces cerevisiae*-Zellen des α-Paarungstyps.

Die jeweiligen Hefezellen bilden kurze Peptide, sog. Hefe-Pheromone, um ihrer Umgebung den eigenen Paarungstyp mitzuteilen. So sezernieren *Saccharomyces cerevisiae*-Hefezellen des Paarungstyps α beispielsweise das Pheromon α-Faktor und *Saccharomyces cerevisiae-*Hefezellen des Paarungstyps a das Pheromon a-Faktor. Die Zellen, z.B. die Hefezellen, besitzen auf ihren Oberflächen Rezeptoren für die Pheromone des jeweils entgegengesetzten Paarungstyps. So sind beispielsweise *Saccharomyces cerevisiae*-Zellen des Paarungstyps a in der Lage, *Saccharomyces cerevisiae*-Zellen des Paarungstyps α in ihrer Umgebung wahrzunehmen und umgekehrt.

Hefe-Pheromone im erfindungsgemäßen Sinn sind außer den in Hefezellen vorkommenden natürlichen Hefe-Pheromonen auch homologe oder modifizierte Peptide oder Peptidanaloga, oder andere organische Verbindungen, die in der Lage sind, an die Pheromon-Rezeptoren von Hefezellen zu binden und diese zu aktivieren. Die DNA-Sequenz, die für das Hefe-Pheromon kodiert, kann entweder ein natürliches, in dem Genom eines Organismus enthaltenes Gen sein, oder eine synthetische Gensequenz, deren Expression zur Produktion eines Hefe-Pheromons oder eines zu einem Hefe-Pheromon homologen Peptids führt, das in der Lage ist, die Pheromonrezeptoren von Hefezellen zu aktivieren.

Die Gene, die in den haploiden Hefezellen für die Synthese des Hefe-Pheromons zuständig sind, sind bevorzugt das *MFα1-* oder *MFα2*-Gen von *Saccharomyces cerevisiae*, welche jeweils für das Pheromon α-Faktor kodieren, oder das *MFA1*-Gen, oder das *MFA2*-Gen von *Saccharomyces cerevisiae*, welche für das Pheromon a-Faktor kodieren.

Erkennen die haploiden Hefezellen mittels des rekombinant exprimierten heterologen Hormonrezeptors in der Probe vorhandene, hormonell wirksame Substanzen, wird die Transkription des durch den Hormonrezeptor regulierbaren Promotors induziert, so dass die haploiden Hefezellen als Antwort auf die hormonell wirksame Substanz das Hefe-Pheromon exprimieren und in die Umgebung sezernieren (Fig. 2 B; Fig. 3 B). Bei der Aktivierung des Androgenrezeptors durch ein Androgen-wirksames Hormon würde beispielsweise das unter die Kontrolle eines *PGK*-Promotor-Derivates, welches über drei Androgen-responsive Elementen verfügt, gestellte Hefe-Pheromon exprimiert und sezerniert werden.

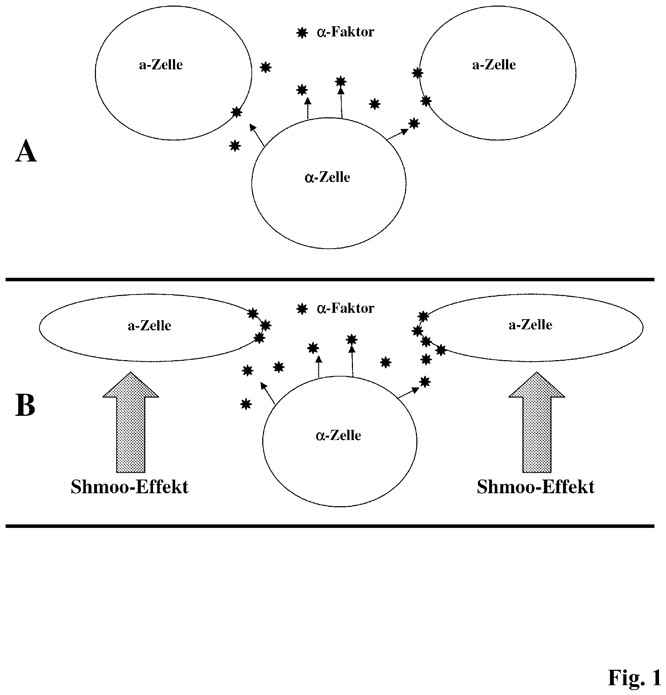

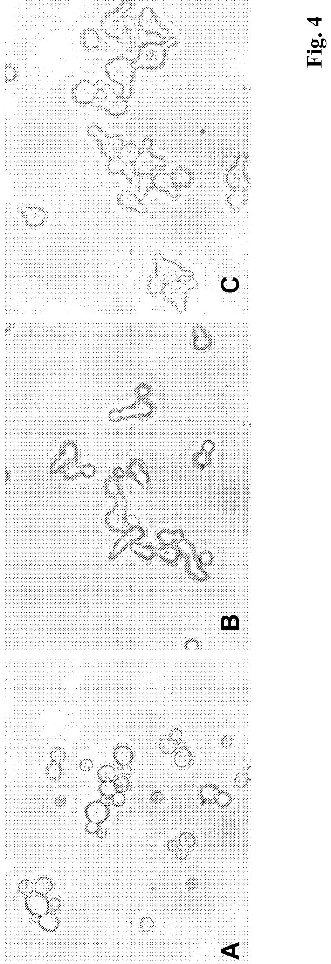

Die erfindungsgemäßen haploiden Hefezellen besitzen auf ihrer Oberfläche Rezeptoren für das sezernierte Hefe-Pheromon (Merkmal 3). Die Aktivierung der Rezeptoren durch das entsprechende Hefe-Pheromon führt zum Ablauf eines umfangreichen genetischen Programms, welches die Veränderung der Transkriptionsaktivität verschiedener Promotoren (z.B. die sehr starke Transkriptionserhöhung (96fach) des *FIG1*-Promotors (Roberts *et al.*, 2000) und eine deutliche Veränderung der Morphologie der Hefezellen (z. B. "Shmoo-Effekt") bedingt (Fig. 4B und C). Die veränderte Morphologie ist lichtmikroskopisch sehr leicht nachweisbar.

Bevorzugt wird daher dasjenige Paarungstyp-spezifische Pheromon verwendet, für welches die haploide Hefezelle selbst Rezeptoren besitzt. Die erfindungsgemäßen haploiden Hefezellen sind dann einheitlich und umfassen alle Merkmale 1 bis 3. Beispielsweise wird in einer haploiden *Saccharomyces cerevisiae*-Hefezelle des a-Typs, die α-Rezeptoren auf ihrer Oberfläche besitzt, das Gen für den α-Faktor unter die Kontrolle eines Promotors gestellt, der durch den heterologen Hormonrezeptor reguliert wird.

Exprimiert diese Hefezelle den heterologen Hormonrezeptor, wird durch die Bindung der hormonell wirksamen Substanz an den Hormonrezeptor die Expression und Sekretion des α-Faktors induziert. Der α-Faktor bindet dann an die entsprechenden Rezeptoren der haploiden Hefezelle und führt zu den oben beschriebenen morphologischen Änderungen, wie beispielsweise Paarungsprojektionen der Zellen.

Alternativ wird in einer haploiden *Saccharomyces cerevisiae*-Hefezelle des α-Typs, die einen heterologen Hormonrezeptor exprimiert, das Gen für den α-Faktor unter die Kontrolle eines Promotors gestellt, der durch den Hormonrezeptor reguliert wird. Diese Hefezelle besitzt Rezeptoren für den a-Faktor und wird nach der Bindung der hormonell wirksamen Substanz und der Expression und Sekretion des a-Faktors ebenfalls die morphologischen Änderungen ausbilden.

Die natürlicherweise für die Hefe-Pheromone kodierenden Gene sind in den genutzten Zellen vorzugsweise ausgeschaltet. Bevorzugt sind die natürlichen Gene *MFα1* und *MFa2* in α-Zellen von *Saccharomyces cerevisiae*-Zellen und die natürlichen Gene *MFa1* und *MFa2* in a-Zellen von *Saccharomyces cerevisiae*-Zellen deletiert. Damit wird vorteilhaft sichergestellt, dass Hefe-Pheromon ausschließlich dann gebildet und sezerniert wird, wenn die nachzuweisende hormonell wirksame Substanz vorhanden ist. Mittel zum Ausschalten von Genaktivität in Zellen sind dem Fachmann bekannt. Dies geschieht beispielsweise durch "Gene-Replacement" (Kaiser et al., 1994). Dadurch lassen sich außerdem vorteilhaft haploide Hefezellen verschiedener Paarungstypen in einer Kultur heranziehen, ohne dass diese durch Hefepaarung in diploide Hefezellen übergehen können.

Der Promotor, der durch das Hefe-Pheromon reguliert wird, ist bevorzugt der *FIG1*-Promotor von *Saccharomyces cerevisiae.* Weitere Pheromon-induzierbare Promotoren kann der Fachmann in einschlägigen Datenbanken, wie z.B. der Saccharomyces Genome Database (SGD) auf der Basis der ermittelten Expressionsprofile finden oder Veröffentlichungen entnehmen.

Die Transkription des *FIG1*-Gens wird nach Inkubation haploider Hefezellen mit Pheromonen des jeweils entgegengesetzten Paarungstyps um das bis zu 97-fache erhöht (Roberts et al., 2000). Es wurde ursprünglich in einem Hefe "Two-Hybrid Screen" zur Identifikation von Pheromon-regulierten Genen identifiziert (Erdman, et al., 1998). Der Name *FIG1* bedeutet "factor induced gene 1". Es handelt sich um ein integrales Membranprotein, welches direkt oder indirekt an der Ca²⁺-Aufnahme in die Zelle beteiligt ist (Muller et al., 2003). Nach Zugabe von α-Faktor zu a-Zellen ist in diesen eine deutliche Zunahme des Proteins nach 60 Minuten nachweisbar (Roberts et al., 2000). Auf Transkriptionsebene wird eine Erhöhung der mRNA Konzentration um mehr als das 97-fache bereits 20 Minuten nach Zugabe des jeweils entgegengesetzten Pheromons zu den Hefezellen beobachtet (Roberts et al., 2000). Der Promotor des *FIG1*-Gens ist daher vorteilhaft durch Hefe-Pheromone des jeweils entgegengesetzten Paarungstyps hochgradig regulierbar. Somit ist die Einwirkung von Hefe-Pheromonen schnell und sensitiv nachweisbar.

Bevorzugt wird als Promotor enthaltender Bereich jeweils ein DNA-Abschnitt verwendet, der bis zu 1000 bp 5'-seitig des *FIG1*-Gens umfasst, oder ein Teilabschnitt dieses DNA-Abschnitts, der in der Lage ist, auf die Anwesenheit eines Hefe-Pheromons hin das unter Kontrolle dieser Sequenz liegende spezifische Gen zu aktivieren oder zu unterdrücken.

Besonders bevorzugt verwendet wird der DNA-Abschnitt, den man durch PCR-Amplifikation des *Saccharomyces*-Genoms unter Verwendung der Primer Fig1-for (SEQ ID No. 2) und Fig1-rev (SEQ ID No. 3) (siehe Tab. 3) erhält.

**Tab. 3: Primer zur Amplifikation des FIG1-Promotors. Die fett gedruckten Buchstaben in den in Tab. 3 aufgeführten Primern grenzen den genutzten Promotorbereich des FIG1-Gens ein. Kursiv sind die Erkennungssequenzen der Restriktionsendonukleasen SacI bzw. SpeI angegeben, welche für die Klonierung genutzt werden.**

| **SEQ ID No.** | **Primer** | **Sequenz (5' → 3')** |
|---|---|---|
| **2** | **Fig1****-for** | |
| **3** | **Fig1****-rev** | |

Im Prozess der Hefe-Paarung induziert der Transkriptionsfaktor Ste12p die Expression von Pheromon-responsiven Genen durch Bindung von Ste12p an sogenannte "pheromon response elements" (PREs) in der Promotorregion induzierbarer Gene (Dolan et al., 1989). Hagen et al. (1991) zeigten, dass tandemartig angeordnete PREs ausreichend sind, um die Pheromonresponsive Expression von haploid-spezifischen Genen in beiden Paarungstypen zu aktivieren. PREs sind 7 bp lange Elemente mit der Konsensus-Sequenz TGAAACA (Kronstad et al., 1987).

Im *FIG1* Promotor wurden drei putative Bindestellen für Ste12p identifiziert (Harbison et al., 2004).

Die Ansprechzeit des *FIG1*-Promotors kann durch eine höhere Anzahl an PREs verkürzt werden. Beispielsweise kann zusätzlich zum oder stellvertretend für den authentischen Aktivatorbereich des Gens ein 139 bp langes Fragment mit den PREs der regulatorischen Region von *FUS1* oder ein einfaches synthetisches Cluster aus PREs genutzt werden (Hagen et al. 1991). So wird vorteilhaft mit einem Reporterkonstrukt aus dem modifizierten *FIG1-*Promotor und einem EGFP-Marker eine höhere Expression des Markergens und bessere Ansprechbarkeit auf geringere Pheromonkonzentrationen erzielt. Ebenfalls wird eine zeitlich schnellere Ansprechbarkeit des Systems erreicht.

Bevorzugt wird in den haploiden Hefezellen, die einen Rezeptor für das Hefe-Pheromon besitzen, daher der Transkriptionsaktivator Ste12p überexprimiert. Die Überexpression von *STE12* führt zu einer verstärkten Expression Pheromon-responsiver Gene, vermittelt durch PREs (Dolan und Fields, 1990). Vorteilhaft wird durch die Überexpression des Transkriptionsaktivators Ste12p auch der Expressionslevel des spezifischen Gens unter der Kontrolle des Pheromon-abhängigen Promotors erhöht.

Um an einzelne PREs zu binden, benötigt Ste12p teilweise weitere Transkriptionsaktivatoren wie den Faktor Mcm1p (Hwang-Shum et al., 1991). Besonders bevorzugt wird daher in den Zellen mit dem Merkmal 3 Mcm1p überexprimiert. Die heterologe Expression dieses Faktors trägt ebenfalls zur Erhöhung der Expression des unter der Kontrolle des Pheromon-abhängigen Promotors stehenden spezifischen Gens bei.

Für ein möglichst zeitnahes Monitoring kann es vorteilhaft sein, die Markerproteine durch das Anfügen von Sequenzen, die zu einem erhöhten "Turn-over" der Proteine führen, zu destabilisieren. Dadurch erhält das als Markerprotein verwendete fluoreszierende Protein eine eingeschränkte Halbwertszeit. Dadurch wird eine rasche Ansprechzeit bei einer Abnahme der Transkription gewährleistet.

Eine solche eingeschränkte Halbwertszeit kann zum Beispiel durch die Veränderung der N-terminalen Aminosäure oder das Einbringen einer Signalsequenz in die Aminosäuresequenz des vom spezifischen Gen kodierten Markerproteins erreicht werden, wodurch die Stabilität des Proteins gesenkt und seine Halbwertszeit verkürzt wird. Bevorzugt verwendet wird für die Destabilisierung des vom Markergen kodierten Proteins eine sogenannte PEST-Domäne, die zu einem raschen Abbau des Proteins durch das Ubiquitin-System der Zelle führt. Solche PEST-Domänen sind aus vielen Proteinen bekannt. Bevorzugt verwendet wird die PEST-Domäne des G1 Cyclins Cln2p aus *Saccharomyces cerevisiae.* Hierfür wird an das 3'-Ende der kodierenden Sequenz des spezifischen Gens die kodierende Sequenz (SEQ ID No. 4) der 178 carboxyterminalen Aminosäuren von Cln2p (SEQ ID No. 5) und ein Stoppcodon angefügt.
Cln2p-Pest-Sequenz (SEQ ID No. 4/5):

Das erfindungsgemäße Verfahren hat den Vorteil, dass das durch die Bindung einer hormonell wirksamen Substanz an den heterologen, nukleären Hormonrezeptor ausgelöste Signal durch die dadurch induzierte Sekretion des Hefe-Pheromons und dessen Wirkung auf die umliegenden Zellen um ein Vielfaches verstärkt werden kann, so dass vorteilhaft die Sensitivität erhöht wird.

Der α-Faktor wird durch die spezifische Protease Bar1p von *Saccharomyces cerevisiae* gespalten und damit inaktiviert. Bar1p wird sezerniert und ist für eine korrekte Paarung der Hefezellen notwendig. MATa-Zellen, bei denen Bar1p inaktiviert ist, zeigen eine deutlich erhöhte Sensitivität gegenüber dem α-Faktor. Um die Sensitivität und Ansprechzeit des Nachweissystems zu erhöhen, werden daher vorteilhafterweise Zellen eingesetzt, bei denen das Bar1-Protein inaktiviert bzw. das entsprechende Gen deletiert wurde (Ballensiefen and Schmitt, 1997; Chan and Otte, 1982; Barkai et al., 1998; Sprague and Herskowitz, 1981).

Weiterhin ist in den haploiden Hefezellen bevorzugt das Protein Fig1p inaktiviert. Hohe lokale Konzentrationen an Hefe-Pheromonen lösen bei Hefezellen Zelltod aus. Durch die Inaktivierung von Fig1p wird dieser Effekt verhindert (Zhang et al., 2006). Dadurch wird vorteilhaft verhindert, dass die Hefezellen durch eine zu hohe Konzentration an Hefe-Pheromon absterben und damit für das Verfahren nicht mehr zur Verfügung stehen (Zhang et al., 2006).

Da die Zellen sich für das erfindungsgemäße Verfahren in einem flüssigen Medium befinden, müssen die zu detektierenden hormonell wirksamen Substanzen ebenfalls in diesem flüssigen Medium vorliegen.

Die haploiden Hefezellen exprimieren erfindungsgemäß mindestens zwei heterologe Gene: Zunächst wird das Gen für den Hormonrezeptor unter der Kontrolle eines konstitutiven Promotors exprimiert. Das unter die Kontrolle eines für einen Hormonrezeptor spezifischen Promotors gestellte Hefe-Pheromon-Gen wird durch die Aktivierung des Hormonrezeptors exprimiert.

Außerdem wird gegebenenfalls ein unter die Kontrolle des Pheromon-Rezeptors gestelltes spezifisches Gen, das für ein Markerprotein kodiert, durch die Sekretion des Hefe-Pheromons exprimiert.

Diese Gene müssen zunächst in die haploide Hefezelle eingebracht werden. Dabei können sie in der haploiden Hefezelle auf einem extrachromosomalen DNA-Molekül vorliegen. Bevorzugt verwendet wird dazu ein Hefe-Expressionsvektor, der bei der Teilung der Hefezelle repliziert und stabil auf die Tochterzellen verteilt wird. Besonders bevorzugt ist ein sogenannter "high copy number" Vektor, der in der Hefezelle in einer großen Anzahl an Kopien vorliegt. Alternativ werden auch Vektoren genutzt, die in geringerer Kopienzahl oder als einzelner Vektor in Hefen vorliegen, z.B. *ARS-CEN-*Vektoren oder künstliche Hefechromosomen (yeast artificial chromosomes).

In einer anderen Ausführungsform wird das betreffende Gen zusammen mit dem regulierbaren Promotor in die chromosomale DNA der haploiden Hefezelle integriert. Dadurch wird vorteilhaft sichergestellt, dass alle Nachkommen der haploiden Hefezelle ebenfalls das Markergen unter Kontrolle des spezifischen Promotors enthalten.

Vorteilhaft für den Einsatz des Verfahrens ist weiterhin, dass Hefen gut getrocknet werden können und so über längere Zeit haltbar sind. So wurde ein kostengünstiges Lufttrocknungsverfahren entwickelt, bei dem zunächst Weizenmehl einer feuchten Hefekultur zugegeben wird und dann eine Lufttrocknung durchgeführt wird. Mit dem Verfahren wurden Überlebensraten der Zellen von bis zu 100% erreicht (Mille *et al.*, 2005). Luftgetrocknete Hefen lassen sich vorteilhaft ohne großen Aufwand transportieren und lagern.

In einer besonderen Ausgestaltung des erfindungsgemäßen Verfahrens können die haploiden Hefezellen auch immobilisiert werden. Als Matrix für die Einbettung eignen sich z. B. Hydrogele aus Calcium-Alginat. Sie besitzen eine poröse Struktur und sind optisch transparent. Diese Eigenschaften ermöglichen einerseits die Interaktion zwischen den immobilisierten Zellen und ihrer Umgebung, andererseits die Detektion von optischen Signalen, die durch die haploiden Hefezellen gebildet werden, z. B. fluoreszierende Proteine. Verfahren zur Einbettung von *Saccharomyces cerevisiae*-Hefezellen in Form von Alginatkügelchen für die Entwicklung von Anwendungen im Bereich Biosensorik/Biotechnologie sind aus der Literatur bekannt (Bleve et al., 2008; Fine et al., 2006). Analog ist eine Immobilisierung der Zellen in dünnen, transparenten Calcium-Alginat-Schichten vorstellbar.

Das erfindungsgemäße Verfahren und die erfindungsgemäßen haploiden Hefezellen lassen sich äußerst vielfältig einsetzen. Bestandteil der Erfindung sind daher auch die Verwendung des erfindungsgemäßen Verfahrens bzw. der erfindungsgemäßen Hefezellen für die Diagnostik, d.h. zum Nachweis von hormonell wirksamen Substanzen in Urin-, Blut- und Serumproben, besonders zum Nachweis von Dopingmitteln in Urinproben. Das erfindungsgemäße Verfahren bzw. die erfindungsgemäßen Hefezellen eignen sich auch für die Verwendung zur Abschätzung der Belastung von Umweltproben mit hormonell wirksamen Substanzen, wie Gewässer- und Gewebeproben, oder zur Einschätzung des Gefahrenpotentials von möglicherweise hormonell wirksamen, synthetischen oder natürlichen Substanzen. Das erfindungsgemäße Verfahren bzw. die erfindungsgemäßen Hefezellen eignen sich dabei besonders für das kostengünstige und schnelle Screening großer Zahlen an Substanzen bzw. komplexer Stoffgemische. Dabei eignen sie sich auch besonders für die Identifizierung potentiell hormonell wirksamer Stoffe für die pharmazeutische Anwendung, beispielsweise aus Pflanzenextrakten oder bei unbekannten Substanzen pflanzlichen oder anderen organischen oder synthetischen Ursprungs. Desweiteren ist das erfindungsgemäße Verfahren auch für eine Anwendung im toxikologischen Screening von Abwässern, Trinkwassern, Verpackungsmaterialien, Lebensmitteln bzw. Nahrungsergänzungsstoffen auf unerwünschte hormonelle Inhaltsstoffe geeignet. Ebenso bevorzugt ist die Verwendung für den ein Einsatz zum Screening auf den Einsatz von Hormonen in der Mast von Tieren, die zur Produktion von Nahrungsmitteln gedacht sind.

Anhand folgender Figuren und Ausführungsformen wird die Erfindung näher erläutert, ohne auf diese beschränkt zu sein.

Dabei zeigen
**Fig. 1****:** Schematische Darstellung des Hefe-Pheromonsystems
**Fig. 2****:** Schematische Darstellung des Verfahrens zum Nachweis hormonell wirksamer Substanzen über die Erfassung morphologischer Änderungen der haploiden Hefezellen
**Fig. 3****:** Schematische Darstellung des Verfahrens zum Nachweis hormonell wirksamer Substanzen über die Erfassung physiologischer Änderungen der haploiden Hefezellen
**Fig. 4****:** Lichtmikroskopische Darstellung des "Shmoo"-Effektes. Links sind sich mitotisch durch Knospung teilende *Saccharomyces cerevisiae*-Zellen des Paarungstyps a gezeigt (Fig. 4 A). Die Aufnahmen in der Mitte (Fig. 4 B) und rechts (Fig. 4 C) zeigen die durch Einwirken eines Hefe-Pheromons (α-Faktor) ausgelösten morphologischen Änderungen ("Shmoo"-Effekt), Vergrößerung 1000fach)
**Fig. 5****:** Sogenannter HALO-Assay unter Verwendung der Hefestämme BY4741 *Δbar1* (im Agar) und BY4741 *Δbar1* p423*GPD-AR1*, p426*PGK-MFα1* (auf Filterplättchen). Verwendung von Dimethylsulfoxid (DMSO) als Negativkontrolle und verschiedenen DHT-Konzentrationen.
**Fig. 6****:** Darstellung des Quotienten aus Fluoreszenzsignal (EGFP) und optischer Dichte für 10⁻¹¹-10⁻⁵ M Dihydrotestosteron (DHT) im Inkubationszeitraum 22-45h bei 30°C für den Hefestamm BY4741 Δ*bar1* p423*GDP-AR1*, p426*PGK-EGFP.*
**Fig. 7****:** Darstellung des Quotienten aus Fluoreszenzsignal (EGFP) und optischer Dichte für 10⁻¹¹-10⁻⁵ M DHT im Inkubationszeitraum 24-39h bei 30°C Hefestamm BY4741 p423*GDP-AR1,* p426*PGK-EGFP.*

### Ausführungsbeispiel 1: Generierung von Hefezellen, die den humanen Androgenrezeptor (AR) exprimieren

Um den Androgenrezeptor konstitutiv in der Hefe zu exprimieren, wurde das Plasmid p423GPD*AR1* konstruiert. Dazu wurde der *AR1*-Leserahmen (GeneID: 367 Accession Nr. NP_000035) in das p423GPD-Plasmid (*Mumberg et al., 1995*) wie folgt kloniert:
Die *AR1*-Gensequenz (2763 bp) wurde mit den Primern hAR1BcuI(SpeI)_f2 (SEQ ID No. 6; TATATAACTAGTATGGAAGTGCAGTTAGGGCT) und hAR1SalI_rev (SEQ ID No. 7; ATATATGTCGACTCACTGGGTGTGGAAATAGATG) PCR-amplifiziert. Durch die in der PCR verwendeten Primer wurden dem *AR1*-Leserahmen am 5'-Ende die *Spe*I-Schnittstelle und am 3'-Ende die Sequenz der *Sal*I-Schnittstelle angefügt. Der PCR-Ansatz wurde zur Weiterverwendung gereinigt.

Der p423*GPD*-Vektor und das gereinigte PCR-Produkt für den *AR1*-Leserahmen wurden mit den Restriktionsenzymen *Spe*I und *Sal*I geschnitten. Anschließend wurden der gereinigte, geschnittenen p423*GPD*-Vektor und das gereinigte, geschnittene PCR-Produkt für den Androgenrezeptor mittels Gelelektrophorese auf einem 1 %igen Agarosegel aufgetrennt. Aus dem Gel wurden die spezifischen Fragmente ausgeschnitten und die darin enthaltene DNA wurde aus dem Agarosegelstück extrahiert. Es schloss sich die Ligation des *AR1*-Fragments und des p423GPD-Plasmids an. Nach erfolgter Elektroporation wurden die Ansätze auf LB-Mediumplatten mit 100 µg/ml Ampicillin ausplattiert. Rekombinante Klone wurden durch einen Kontrollverdau isolierter Plasmid-DNA mit dem Restriktionsenzym *Kpn*I identifiziert.

Um die korrekte Lage des *AR1*-Leserahmens in der MCS überprüfen zu können, wurde die Plasmid-DNA sequenziert. Die Sequenzierung mit dem GPDpromseqfor-Primer (SEQ ID No. 8; CGGTAGGTATTGATTGTAATTCTG) ergab, dass das *AR1*-Gen an der richtigen Stelle in der MCS integriert wurde.

### Ausführungsbeispiel 2: Generierung von Hefezellen, die ein Gen für den α-Faktor unter der Kontrolle eines Androgen-induzierbaren Promotor beinhalten

Der *MFα1*-Leserahmen, welcher für den α-Faktor kodiert, wurde hinter einen Androgen-sensitiven Promotor in das Plasmid p426PGK (siehe unten) kloniert.

Der Promotor wurde im Androgen-induzierbaren Hefe-Expressionstamm PGKhAR genutzt.

Der Hefestamm PGKhAR wurde von Purvis *et al.* (1991) beschrieben. Bei diesem Stamm wurde die DNA-Sequenz für den humanen Androgenrezeptor stabil in das Genom integriert. Der Androgenrezeptor wird konstitutiv exprimiert. Zusätzlich besitzt der Hefestamm ein Plasmid, welches ein Derivat des *PGK*-Promotors mit drei Androgen-responsiven Elementen und das *lac*Z-Gen als Reportergen trägt. Da es sich bei dem *PGK*-Promotor um ein Derivat handelt, wird dieser nicht konstitutiv in den Hefezellen exprimiert. In Anwesenheit von Androgenen bindet der durch die Ligandenbindung aktivierte Androgenrezeptor am *PGK-*Promotor-Derivat und initiiert so die Transkription des Reportergens *lac*Z. Die Expression des Reportergens erfolgt in hohem Maße. Nach der Translation wird die β-Galactosidase von der Hefezelle sezerniert. Die Aktivität des Enzyms kann photometrisch bei 540 nm gemessen werden, da bei der enzymatischen Substratumsetzung ein farbiges Produkt entsteht (Sohoni & Sumpter, 1998; Routledge & Sumpter 1996).
Die Klonierung des *MFα*1-Leserahmens wurde wie folgt realisiert: Zur Vervielfältigung des *MFα*1-Leserahmens (GeneID:855914 Acc. Nummer X01581) wurde das Primer-Paar MFalpha1SpeI_for/MFalpa1BamHI_re (SEQ ID No. 9; TATATTACTAGTATGAGATTTCCTTCAATTTTTACTG/ SEQ ID No. 10 TATATTGGATCCTTAGTACATTGGTTGGCCG) und als Template das Plasmid p426*GPDMFα*1 genutzt. p426*GPDMFα*1 basiert auf dem Vektor p426*GPD* (Mumberg et al., 1995), in den der *MFα*1-Leserahmen über die *Eco*RI*-* und *Sal*I-Restriktionsschnittstellen eingefügt wurde.

Durch die bei der PCR gewählten Primer wurden dem *MFα*1-Gen am 5'-Ende die Sequenz einer *Spe*I-Schnittstelle und am 3'-Ende die Nukleotidabfolge einer *Bam*HI-Schnittstelle angefügt. Das PCR-Produkt wurde mittels Agarosegelelektrophorese analysiert und anschließend aufgereinigt.

Der *MFα*1-Leserahmen wurde in den Vektor p426*PGK* kloniert, der auf dem Vektor p426*ADH* basiert (Mumberg et al., 1995). Der Vektor p426*ADH* wurde zunächst mit dem Restriktionsenzym *Sac*I verdaut, um nachfolgend den *ADH1*-Promotor zu entfernen. Da *Sac*I auch im *PGK*-Promotor eine Schnittstelle besitzt, wurden die überhängenden 3'-Enden des Vektors mittels S1-Nuklease abgebaut. Dies führte zur blunt end-Generierung. Das *PGK-*Promotor-Derivat (SEQ ID No. 1) wurde per PCR amplifiziert. Als Template diente das von Sohoni & Sumpter (1998) beschriebene Plasmid, welches ein Derivat des *PGK*-Promotors mit 3 AREs trägt. Durch den Verdau des PCR-Produkts mit der Restriktionsendonuklease *Dra*I wurde eine Ligation mit den blunt ends des geschnittenen p426*ADH-*Plasmids ermöglicht. Anschließend wurden das *PGK* PCR-Produkt und das bearbeitete p426*ADH-*Plasmid mit *Spe*I verdaut. Somit wurde der *ADH1*-Promotor aus dem Plasmid geschnitten und kompatible überhängende Enden am PCR-Produkt generiert. Es folgte die Ligation des Derivates des *PGK*-Promotors mit 3 AREs (Sohoni & Sumpter, 1998) (SEQ ID No. 1) in das p426-Plasmid.

Das gereinigte PCR-Produkt für das *MFα*1-Gen und der p426*PGK*-Vektor wurden mit den Restriktionsenzymen *Bam*HI und *Spe*I verdaut. Für die Ligation wurde ein molares Verhältnis Vektor:Insert von ungefähr 1:9 gewählt. Die Ligation erfolgte bei 4 °C über Nacht.

Der Ligationsansatz für das Plasmid p426*PGKMFα*1 wurde in den *E. coli* Stamm DH10B transformiert. Die Transformationsansätze wurden auf LB-Platten mit 100 µg/ml Ampicillin ausplattiert und über Nacht bei 37°C kultiviert.

Zur Identifizierung rekombinanter Plasmide wurde eine PCR der Transformanden mit dem Primer-Paar MFalpha1SpeI_for/ MFalpa1BamHI_re durchgeführt. Es wurde in einem rekombinanten Plasmid mit dem MFfor-Primer (SEQ ID No. 11; TCGTAGTTTTTCAAGTTCTTAGATGC) der vollständige *MFα*1-Leserahmen sequenziert. Die Sequenzierung ergab, dass keine Mutationen vorlagen. Der so generierte Vektor p426*PGKMFα*1 konnte für folgende Versuche verwendet werden.

### Ausführungsbeispiel 3: Inaktivierung der chromosomalen Gene, welche für Hefe-Pheromone kodieren

Vorteilhafterweise werden für das erfindungsgemäße Verfahren Hefen genutzt, deren authentische Expression von Hefe-Pheromonen inaktiviert wurde. Hierzu können die entsprechenden Gene deletiert werden. Beispielhaft ist die Inaktivierung der Gene *MFa1* und *MFa2,* die beide für den α-Faktor kodieren, beschrieben. Derart veränderte Hefezellen sind nicht mehr in der Lage, endogen den α-Faktor zu bilden.

Für die Deletion der Leserahmen von *MFα1* und *MFa2,* z.B. im α-Hefestamm BY4742 (*MATα*, *his3Δ1*, *leu2Δ0*, *lys2Δ0*, *ura3Δ0*), werden die Markerkassetten *natMX6* bzw. *hphMX6* verwendet, welche Resistenzen gegen die Antibiotika Nourseothricin bzw. Hygromycin B vermitteln. Die *natMX6*-Kassette wird in einer SFH-PCR mittels der Primer SEQ ID No. 12 und SEQ ID No. 13 aus Tab. 4 amplifiziert. Die 5'-Bereiche der Primer (je 50 Basen) sind homolog zu den flankierenden Sequenzen des *MFα1*-Leserahmens im Genom von *Saccharomyces cerevisiae.* Die 3'-Bereiche der Primer (20 bp) sind homolog zu den Enden der *natMX6*-Kassette. Als DNA-Template für die SFH-PCR dient das Plasmid pFA6a-natMX6 (Hentges et al., 2005). Anschließend werden Hefezellen mit dem SFH-Fragment transformiert. Transformanden, bei denen das Fragment über doppelte homologe Rekombination stabil in das Genom integriert ist, werden auf Nourseothricin-haltigem Medium selektiert und die korrekte Integration der Deletionskassette mittels diagnostischer PCR bestätigt. Danach erfolgt die Deletion des Leserahmens von *MFa2* im erzeugten *Δmfα1-*Hefestamm. Hierzu wird analog zur ersten Deletion ein SFH-Fragment mit den Primern SEQ ID No. 14 und SEQ ID No. 15 (siehe Tab. 4) und der hphMX6-Kassette (DNA-Template pFA6a-hphMX6, Hentges et al., 2005) amplifiziert und in *Δmfα1*-Hefezellen transformiert. Die 5'-seitigen Bereiche der Primer sind homolog zu den flankierenden Sequenzen des *MFα2*-Leserahmens im Genom von *Saccharomyces cerevisiae.* Die Selektion positiver Transformanden erfolgt auf Hygromycin B-haltigem Medium und die korrekte Integration der Hygromycin-Resistenzkassette im *Δmfα1-Δmfα2*-Hefestamm wird mittels diagnostischer PCR überprüft.

**Tab. 4: Primer für die Deletion der Leserahmen von MFα1 und MFα2 von Saccharomyces cerevisiae. Die unmarkierte Primersequenz kennzeichnet Bereiche, die homolog zur genomischen DNA von Saccharomyces cerevisiae sind. Zur Deletionskassette homologe Bereiche sind fett markiert.**

| **SEQ ID No.** | **Bezeichnung** | **Sequenz (5' → 3')** |
|---|---|---|
| 12 | MFalp1_F2 | |
| 13 | MFalp1_R1 | |
| 14 | MFalp2_F2 | |
| 15 | MFalp2_R1 | |

Die natürlichen Gene *MFa1* und *MFa2,* welche für das Pheromon a-Faktor kodieren, werden analog der obigen Vorgehensweise inaktiviert. Die Herstellung von Stämmen, bei denen alle endogen für die Hefe-Pheromone kodierenden Gene deletiert sind, erfolgt im genetischen System Hefe vorteilhafterweise mittels Tetradenanalyse. Z.B. können in der Kreuzung zweier jeweils einzeln Hygromycin-resistenter Stämme in der Tetrade des so genannten "Nicht Parentalen Dityps" die Doppelmutanten eindeutig identifiziert werden. Entsprechend werden Stämme hergestellt, deren Gene *MFa1, MFa2, MFα1* und *MFa2* deletiert sind.

### Ausführungsbeispiel 4: Generierung von Hefezellen zum Nachweis androgener Substanzen mittels Zell-morphologischer Veränderungen ("Shmoo"-Effekt)

Ein Hefestamm, z.B. W303 (*Mat a, ade2-1, his3*□*1, his3-15, leu2-3, leu2-112, trp1-1, ura3-1),* bei dem das *BAR1*-Gen deletiert wurde, wird mit den Plasmiden p426PGKMFα1 und p423GPDAR1 transformiert. Das Plasmid p426PGKMFα1 trägt zur Selektion in Hefe das *URA3*-Gen. Aufgrund von genomischen *ura3*-Mutationen Uracil-bedürftige Hefen sind nach Transformation mit dem Plasmid nicht mehr auf die Zugabe von Uracil angewiesen. Das Plasmid p423GPDAR1 kann über das *HIS3* Gen selektiert werden. Entsprechende Histidin- und Uracil-prototrophe Transformanden werden selektiert. Die Expression des Androgenrezeptors in den Zellen wird mittels Western-Blot Analysen verifiziert. Die Induktion morphologischer Änderungen durch den α-Faktor wird mikroskopisch dokumentiert.

Der so hergestellte Stamm wird mit Stämmen des Paarungstyps a gemischt, welche mit dem Vektor p426FIG1-EGFP ausgestattet sind (siehe Ausführungsbeispiel 6). Die Induktion der Expression und Sekretion des α-Faktors in den transformierten W303-Zellen führt in den mit p426FIG1-EGFP transformierten Zellen zur verstärkten Bildung von EGFP, was entsprechend ausgelesen werden kann. Ggf. können solche Mischkulturen von verschieden transformierten Zelltypen zur Signalverstärkung genutzt werden.

### Ausführungsbeispiel 5: Inaktivierung des BAR1-Gens in Hefezellen zur Erhöhung der Sensitivität des Nachweisverfahrens

Für das erfindungsgemäße Verfahren werden vorteilhaft Hefen eingesetzt, bei denen die Protease Bar1p inaktiviert ist, welche Rezeptor-gebundenen α-Faktor an der Zelloberfläche von *Saccharomyces cerevisiae* abbaut. Vorzugsweise wird dazu der *BAR1*-Leserahmen mittels der Markerkassette *bleMX6* deletiert. Die Kassette vermittelt eine Resistenz gegen das Antibiotikum Phleomycin.

Dazu wird in einer SFH-PCR mit den Primern SEQ ID No. 16 und Seq.ID-Nr. 17 in Tab. 5 die *bleMX6*-Markerkassette von dem Plasmid pFA6-bleMX6 (Hentges et. al., 2005) amplifiziert. Die 50 Basen im 5'-Bereich der beiden Primer sind homolog zu den flankierenden Sequenzen des *BAR1*-Leserahmens, die 20 Basen des 3'-Bereichs der beiden Primer sind homolog zu den Flanken der *bleMX6*-Kassette. Anschließend werden Hefezellen, z.B. *Saccharomyces cerevisiae*-Zellen mit dem Genotyp *MAT*a *ΔMFa1*, *ΔMFa2*, mit dem SFH-Fragment transformiert, um den *BAR1*-Leserahmen durch doppelte homologe Rekombination mit der Markerkassette zu deletieren. Die Selektion von positiven Transformanden erfolgt auf Phleomycin-haltigem Medium. Die korrekte genomische Integration wird mittels diagnostischer PCR verifiziert.

Die Herstellung weiterer Hefestämme mit deletiertem *BAR1* erfolgt analog zu Anwendungsbeispiel 4 mit der beschriebenen Tetradenanalyse.

**Tab. 5: Primer für die Deletion des Leserahmens der Protease Bar1p in Saccharomyces cerevisiae mit einer Markerkassette ble6MX. Die unmarkierte Primersequenz kennzeichnet Bereiche, die homolog zur genomischen DNA von Saccharomyces cerevisiae sind. Zur bleMX6-Deletionskassette homologe Bereiche sind fett markiert.**

| **SEQ ID No.** | **Bezeichnung** | **Sequenz (5' → 3')** |
|---|---|---|
| 16 | deltaBar1_F2 | |
| 17 | deltaBAR1_ R1 | |

### Ausführungsbeispiel 6: Erzeugung von haploiden Hefezellen, die ein Markerprotein unter der Kontrolle eines durch ein Hefe-Pheromon regulierbaren Promotors enthalten

Die im gleichen Ansatz als Zellen des zweiten Typs vorliegenden *Saccharomyces cerevisiae-*Hefezellen des Paarungstyps a sind dahingehend modifiziert, dass sie den für EGFP kodierenden Leserahmen unter der Kontrolle des *FIG1*-Promotors beinhalten.

Dazu wurden 1000 bp 5'-seitig des offenen Leserahmens von *FIG1* unter Verwendung der Primer Fig1-for (SEQ ID No. 18) und Fig1-rev (SEQ ID No. 19) (siehe Tab. 6) PCR-amplifiziert, aufgereinigt, mit den Restriktionsendonukleasen *Sac*I und *Spe*I geschnitten und in den *Saccharomyces cerevisiae* Vektor p426 kloniert. Der so entstandene Vektor (p426FIG1) wurde mit den Enzymen *Sal*I und *Eco*RI geschnitten.

Der für EGFP kodierende Leserahmen wurde mittels der Primer EGFPEcofor und EGFPSalrev PCR amplifiziert und das 744 bp große Fragment mit den Enzymen *Sal*I und *Eco*RI geschnitten, aufgereinigt und zur Ligation in den Vektor p426FIG1 genutzt.

**Tab. 6: Primer zur Amplifikation des EGFP-Gens. Die fett gedruckten Buchstaben grenzen den kodierenden Leserahmen des EGFP-Gens ein. Kursiv sind die Erkennungssequenzen der Restriktionsendonukleasen EcoRI bzw. SalI angegeben, welche für die Klonierung in den Vektor p426FIG1 genutzt werden.**

| **SEQ ID No.** | **Primer** | **Sequenz** |
|---|---|---|
| **18** | EGFP Ecofor | |
| **19** | EGFPSalrev | |

Die DNA-Sequenz des klonierten Leserahmens wurde durch DNA-Sequenzanalyse verifiziert. Damit stand der Vektor p426FIG1-EGFP für die Transformation von Hefezellen zur Verfügung.

Erreicht nach Induktion des spezifisch regulierten Promotors der dann gebildete und sezernierte α-Faktor umliegende *Saccharomyces cerevisiae*-Hefezellen des Paarungstyps a, wird in diesen Zellen die Transkription des für GFP kodierenden Leserahmens mittels des *FIG1*-Promotors stark induziert. Dies resultiert in einer grünen Fluoreszenz der Hefezellen, welche sensortechnisch ausgelesen werden kann. Die Intensität der grünen Fluoreszenz kann proportional zur Zahl der die α-Zelle umgebenden a-Zellen erhöht werden.

### Ausführungsbeispiel 7: HALO-Assay

Unter Verwendung des erfindungsgemäßen Verfahrens wird ein modifizierter HALO-Assay zur Bestimmung der Hormon-/Androgenkonzentration einer Lösung eingesetzt.

Der sogenannte HALO-Assay wird üblicherweise als pheromonbasiertes Testsystem auf Agarplatten zur Bestimmung des Paarungstyps eines Hefestammes eingesetzt. Je nach Paarungstyp bilden und sekretieren *S. cerevisiae*-Stämme den a- oder α-Faktor als Pheromon. Die Erkennung des Peptids des jeweils anderen Paarungstyps, über Oberflächenrezeptoren führt unter anderem zum Arrest der Zellen in der G₁-Phase des Zellzyklus, d. h. dass diese Zellen nicht oder sehr schlecht wachsen.

Zum Nachweis der Androgenkonzentration einer Lösung werden zunächst die Hefestämme BY4741 *Δbar1* in YPD-Medium und BY4741 *Δbar1* p423*GPD-AR1*, p426*PGK-MFα1* in WO-Medium mit Leucin und Methionin über Nacht bei 30 °C unter Schütteln inkubiert. Der Hefestamm BY4741 *Δbar1* soll die Plateauphase der Wachstumskurve erreichen. Für den BY4741 *Δbar1* p423*GPD-AR1*, p426*PGK-MFα1-*Hefestamm ist eine optische Dichte von OD₆₉₀ₙₘ = 1 ausreichend.

Es wird YPD-Agar (0,5 %) erwärmt und auf 55 °C abgekühlt. Zu je 25 ml flüssigem YPD-Agar werden 10 µl des BY4741 *Δbar1*-Hefestammes gegeben. Anschließend polymerisiert der Agar in Petrischalen aus. Währenddessen wird die Übernachtkultur des BY4741 *Δbar1* p423*GPD-AR1*, p426*PGK-MFα1*-Hefestammes abzentrifugiert und das Pellet in 1,5 ml Medium aufgenommen. Von dieser Zellsuspension werden jeweils 100 µl mit verschiedenen DHT-Konzentrationen oder einem anderen Hormon versehen. Als Negativkontrolle dient Dimethylsulfoxid (DMSO). 20 µl der hormonhaltigen Zellsuspension werden auf sterile Filterplättchen gegeben. Diese werden auf die erstarrten Agarplatten gelegt. Die Inkubation der Agarplatten erfolgt bei 30 °C im Brutschrank über 48 h.

Wird der BY4741 *Δbar1* p423*GPD-AR1*, p426*PGK-MFα1*-Hefestamm mit einem Androgen inkubiert, so erkennt der konstitutiv exprimierte Androgenrezeptor das Androgen und induziert die Expression des *MFα1*-Gens. Der gebildete α-Faktor wird sekretiert und vom BY4741 *Δbar1*-Hefestamm über Oberflächenrezeptoren erkannt, da dieser den Paarungstyp a aufweist. Somit kommt es in Anwesenheit von Androgenen zur Wachstumsinhibition, welche an Hemmhöfen auf der Agarplatte erkennbar ist.

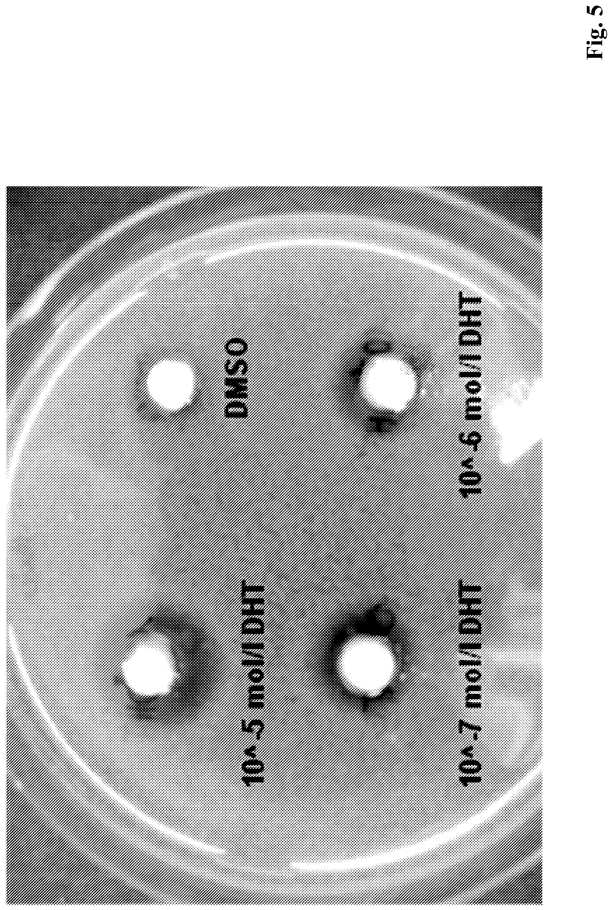

Die Ergebnisdokumentation erfolgt durch das Fotografieren der Agarplatten und das Ausmessen der entstandenen Hemmhöfe. Beispielhaft soll an dieser Stelle die folgende Abbildung (vgl. **Fig. 5**) gezeigt werden. Es ist ein deutlicher Unterschied hinsichtlich der Hemmhofgröße zwischen der Negativkontrolle und den verschiedenen Androgenkonzentrationen erkennbar. Somit kann mittels des HALO-Assays eine qualitative und eine semi-quantitative Aussage über den Androgengehalt einer Lösung getroffen werden.

### Ausführungsbeispiel 8: Analyse androgener Eigenschaften von Substanzen mittels eines Reportergens (EGFP) in Saccharomyces cerevisiae

Die zu verwendenden Hefestämme besitzen identische Auxotrophien, um bei gemeinsamer Inkubation einen Plasmidverlust zu vermeiden. In diesem Fall werden die Leucin- und Methioninauxotrophie der Hefestämme genutzt.

Als Positivkontrolle wurde zunächst ein Hefestamm getestet, der sowohl den Hormonrezeptor konstitutiv exprimiert als auch das EGFP-Gen unter der Kontrolle des Androgen-sensitiven Promotors enthält. Dazu werden die Hefestämme BY4741 *Δbar1* p426*PGK-EGFP*, p423*GPD-AR1* und **BY4741 *Δbar1* p426*PGK*, *p423GPD*** unter Zugabe verschiedener Konzentrationen des Androgens DHT (Dihydrotestosteron) kultiviert. DHT wird von den Hefen aufgenommen und bindet intrazellulär am Androgenrezeptor Isoform1(AR1), der unter der Kontrolle des GPD-Promotors konstitutiv gebildet wird. Der Rezeptor-Ligand-Komplex lagert sich wiederum an den androgen-responsiven Elementen im *PGK*-Promotor des p426*PGK*-*EGFP*-Plasmids an und induziert die Expression des *EGFP*-Gens. Somit kann in Anwesenheit von androgen-wirksamen Substanzen ein Fluoreszenzsignal ohne Verstärkungseffekt detektiert werden.

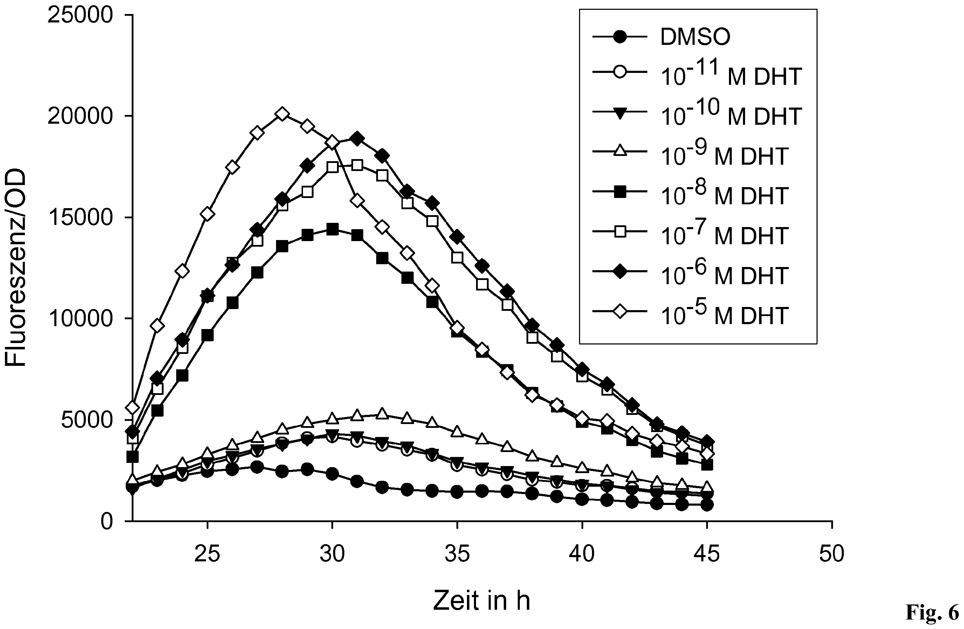

Zur Überprüfung der Funktionalität des BY4741 *Δbar1* p426*PGK-EGFP*, p423*GPD-AR1-*Hefestammes wurde eine Inkubation der Hefezellen in einer 96well-Platte durchgeführt. Hierfür wurden 5x10⁴ Zellen/well in 100 µl W₀-Medium mit Leucin und Methionin in Anwesenheit verschiedener Androgenkonzentrationen ***von 0 bis 45 h*** bei 30°C inkubiert. Die Messung des EGFP-Signals erfolgte stündlich im Zeitraum ***von 22 h - 45 h*** der Inkubation bei einer Anregungswellenlänge von 485 nm und Emissionswellenlänge von 535 nm. Als Referenz wird die Absorption bei 690 nm bestimmt. Beispielhaft wird an dieser Stelle das EGFP-Signal in Anwesenheit verschiedener DHT-Konzentrationen dargestellt (vgl. **Fig. 6**).

Ein deutliches EGFP-Signal konnte ab einer DHT-Konzentration von 10⁻⁸ M beobachtet werden. Niedrigere Androgenkonzentrationen unterschieden sich nicht wesentlich von der Negativkontrolle (DMSO = Dimethylsulfoxid). Als optimaler Messzeitpunkt kann eine Inkubationszeit von 30 h festgehalten werden. Das Experiment zeigte, dass der Hefestamm BY4741 *Δbar1* p426*PGK-EGFP*, p423*GPD-AR1* mit DHT induziert werden kann ***und somit für Versuche des Verstärkersystems eingesetzt werden kann.*** Weitere Messungen zeigten, dass die EGFP-Bildung auch durch weitere androgen-wirksame Substanzen angeregt werden kann.

Als Negativkontrolle werden zunächst die Hefestämme BY4741 *Δbar1* p423*GDP-AR1*, p426*PGK* und BY4741 *Δbar1* p426*FIG1**-EGFP*, *p423 GPD* in Anwesenheit verschiedener Androgenkonzentrationen miteinander inkubiert. Der Hefestamm BY4741 *Δbar1 p423GDP-AR1*, *p426PGK* erkennt androgen-wirksame Substanzen, da der Androgenrezeptor konstitutiv gebildet wird. Dieser Hefestamm produziert jedoch keinen α-Faktor, da das *MFα1*-Gen fehlt. Somit kann die *EGFP*-Expression im BY4741 *Δbar1* p426*FIG1**-EGFP*, *p423GPD-*Hefestamm nicht induziert werden. Es sollte kein EGFP-Signal messbar sein. Anhand dieser Kontrolle kann eine α-Faktor-unabhängige Induktion der *EGFP*-Expression ausgeschlossen werden.

Eine weitere Negativkontrolle stellt die gemeinsame Kultivierung der Hefestämme BY4741 *Δbar1 p423 GPD, p426PGK-MFal* und BY4741 *Δbar1* p426*FIG1**-EGFP*, *p423 GPD* dar. Anhand dieser Kontrolle kann eine Induktion der *MFα1*-Expression unabhängig von der Bindung des Rezeptor-Ligand-Komplexes überprüft werden. Durch das fehlende *AR1*-Gen liegt kein Androgenrezeptor in den Hefezellen vor, an den androgen-wirksame Substanzen binden können. Somit kann die Expression des *MFα1*-Gens nicht induziert werden. Aufgrund des fehlenden α-Faktors ist die Bildung von EGFP nicht möglich.

Zur Anwendung des Verstärkersystems, bei dem man sich zweier verschiedener haploider Hefezellen bedient, werden die Hefestämme BY4741 *Δbar1 p426PGK-MFa1*, p423*GPD-AR1* (Sensorstamm) und BY4741 *Δbar1* p426*FIG1**-EGFP*, p423*GDP* (Reporterstamm) in Anwesenheit verschiedener Androgenkonzentrationen miteinander inkubiert. Gemäß dem erfindungsgemäßen Verstärkersystem kommt es in Anwesenheit von androgen-wirksamen Substanzen zur Bildung von EGFP.

Die Durchführung des Experiments ist neben den BY4741 *Δbar1*-Hefestämmen auch mit BY4741-, BY4742 *Δmfα1 Δmfα2-* und BY4742 *Δbar1 Δmfα1 Δmfα2*-Hefestämmen, welche die oben genannten Plasmidkombinationen tragen, möglich.

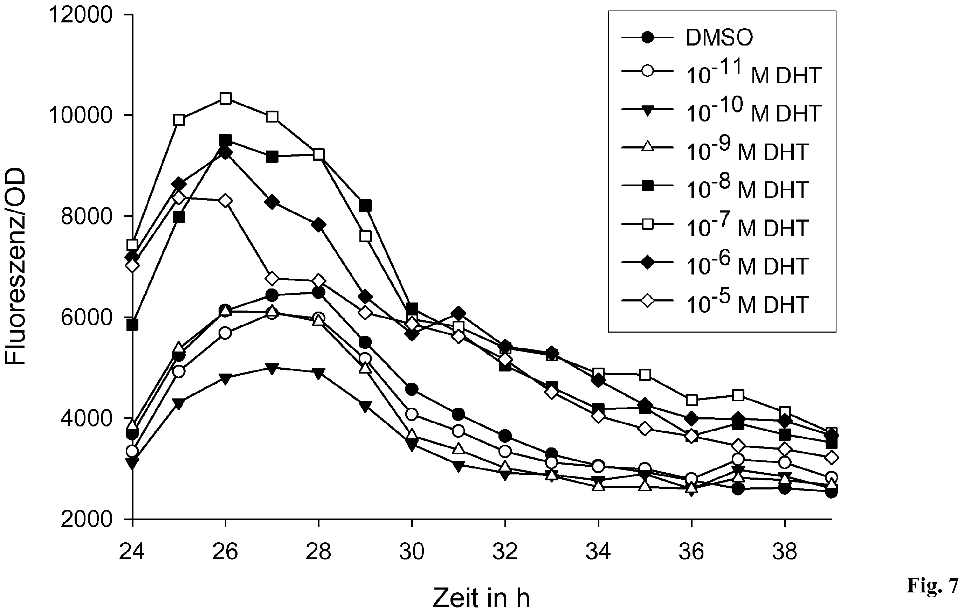

Die Funktionalität des BY4741 p426*PGK-EGFP*, p423*GPD-AR*1-Hefestammes wurde bereits für verschiedene androgen-wirksame Substanzen nachgewiesen. Die Durchführung entspricht der oben genannten Beschreibung zur Testung des BY4741 *Δbar1* p426*PGK-EGFP*, p423*GPD*-*AR1*-Hefestammes. Die folgende Abbildung zeigt beispielhaft das Ergebnis für DHT (vgl. Fig. 7).

Ein erstes messbares EGFP-Signal konnte bei 10⁻⁸ M DHT beobachtet werden. Geringere Androgenkonzentrationen unterschieden sich nicht von der Negativkontrolle (DMSO). Der optimale Messezeitpunkt liegt bei 26 h Inkubationszeit, da hier das höchste EGFP-Signal festgestellt wurde. Zusammenfassend ist zu sagen, dass die *EGFP*-Expression im BY4741 p426*PGK-EGFP*, p423*GPD*-*AR1*-Hefestamm durch DHT induziert werden kann und somit auch der Androgenrezeptor in diesen Hefezellen funktionell vorliegt. Weitere (hier nicht dargestellte) Messungen zeigen, dass neben DHT auch andere androgen-wirksame Substanzen die EGFP-Bildung bewirken.

### Ausführungsbeispiel 9: Shmoo-Effekt

Die Erkennung des α-Faktors (Hefepheromon) über Oberflächenrezeptoren bewirkt unter anderem ein gerichtetes Auswachsen (Shmoo-Effekt) der Hefezellen hin zur Quelle des Pheromons. Unter natürlichen Bedingungen dient dies zur anschließenden Fusion mit der Hefezelle des entgegengesetzten Paarungstyps.

Zur Detektion androgen-wirksamer Substanzen mittels Shmoo-Phänotyp können zwei Versuchsansätze genutzt werden. Der Nachweis der Androgenität einer Substanz kann durch ein Einzellsystem oder mittels eines Verstärkersystems erbracht werden.

Zur Nutzung des Einzellsystems wird zunächst eine Übernachtkultur des Hefestammes BY4741 *MATa* p423*GPD-AR1*, p426*PGK-MFα1* in W₀-Minimalmedium unter Zusatz von Leucin und Methionin angesetzt. Die Inkubation erfolgt bei 30 °C. Die Hefestämme BY4741 *MATa Δbar1* p423*GPD-AR1*, p416*PGK-MFα1*; BY4742 *MATα Δbar1 Δmfα1 Δmfα2* p423*GPD-AR1*, p426*PGK-MFα1* und BY4742 *MATα Δmfα1 Δmfα2* p423*GPD-AR1*, p426*PGK-MFα1* können ebenfalls eingesetzt werden. Beispielhaft wird der Versuchsaufbau unter Verwendung des Hefestammes BY4741 *MATa* p423*GPD-AR1*, p426*PGK-MFα1* erläutert.

Aus der Übernachtkultur des Hefestammes BY4741 *MAT*a p423*GPD-AR1*, p426*PGK-MFα1* wird nun frisches W₀-Minimalmedium mit dem Zusatz von Leucin und Methionin angeimpft. Zur Positivkontrolle wird gereinigter α-Faktor gegeben. Es kommt zum gerichteten Auswachsen der Hefezellen, da der α-Faktor über die Oberflächenrezeptoren des a-Hefestammes wahrgenommen werden kann. Anhand dieser Kontrolle kann die androgen-unabhängige Induktion der Hefezellen überprüft werden.

Als Negativkontrolle wird DMSO (Dimethylsulfoxid) bzw. das Lösungsmittel der androgen-wirksamen Substanz zum Medium gegeben. In Abwesenheit einer androgen-wirksamen Substanz kann sich kein Rezeptor-Ligand-Komplex bilden und folglich die Expression des *MFα1*-Gens durch Anlagerung an den androgen-responsiven Elementen des *PGK*-Promotors nicht bewirken. Da kein α-Faktor gebildet werden kann, tritt kein Shmoo-Phänotyp auf. Somit kann eine androgen-unabhängige Induktion der *MFα1*-Expression ausgeschlossen werden.

Zur Analyse der Androgenität einer Testsubstanz werden die Hefezellen mit verschiedenen Konzentrationen dieser Testsubstanz inkubiert. Die Inkubation aller Ansätze erfolgt bei 30°C. Die Auswertung des Experiments erfolgt mittels eines Lichtmikroskops. Es werden stündlich Zellen aus jedem Ansatz entnommen und mittels einer Neubauer-Zählkammer ausgezählt. Hierbei werden die Gesamtzellzahl und der Anteil an Hefezellen mit Shmoo-Phänotyp bestimmt.

Zur Nutzung des Verstärkersystems werden ebenfalls Übernachtkulturen aller zu verwendenden Hefestämme, wie oben beschrieben, angefertigt. Beispielhaft wird der Versuchsaufbau mit den folgenden Hefestämmen BY4741 *MAT*a p423*GPD-AR1*, p426*PGK-MFα1*; BY4741 *MAT*a p423*GPD-AR1*, p426*PGK*; BY4741 *MAT*a p426*PGK-MFα1*, *p423 GPD;* BY4741 *MATa* p426*FIG1**-MFα1*, *p423 GPD* und BY4741 *MAT*a p426*FIG1*, p423GPD beschrieben. Die Durchführung ist auch mit den Hefestämme BY4741 *MATa Δbar1*; BY4742 *MATα Δmfα1 Δmfα2* und BY4742 *MATα Δbar1 Δmfα1 Δmfα2*, welche die genannten Plasmidkombinationen tragen, möglich. Ebenso kann das Plasmid p416*PGK-MFα1* verwendet werden.

Auch für das Verstärkersystem wird W₀-Minimalmedium unter Zusatz von Leucin und Methionin mit den Übernachtkulturen angeimpft.

Als Positivkontrolle werden die Hefestämme BY4741 *MAT*a p423*GPD-AR1*, p426*PGK-MFα1* (Sensorstamm) und BY4741 *MAT*a p426*FIG1*, p423*GPD* (Reporterstamm) mit verschiedenen Konzentrationen eines Androgens (bevorzugt DHT) kokultiviert. Das Androgen bildet mit dem konstitutiv gebildeten Androgenrezeptor Isoform1 (AR1) einen Rezeptor-Ligand-Komplex, welcher die Expression der *MFα1*-Gens im Sensorstamm induziert. Der α-Faktor wird sekretiert und bewirkt einen Shmoo-Phänotyp der Hefezellen. Ein Verstärkungseffekt bleibt aus, da hinter den Pheromon-responsiven *FIG1*-Promotor des Reporterstammes kein *MFα1*-Gen geschalten ist.

Als eine Negativkontrolle dient die gemeinsame Kultivierung der Hefestämme BY4741 *MAT*a p423*GPD-AR1*, p426*PGK* (Sensorstamm) und BY4741 *MAT*a p426*FIG1**-MFα1*, *p423GPD* (Reporterstamm) in Anwesenheit verschiedener Androgenkonzentrationen (bevorzugt DHT). Es kann zwar ein Rezeptor-Ligand-komplex gebildet werden, dieser induziert aber keine α-Faktor-Produktion, da das *MFα1*-Gen im Sensorstamm fehlt. Folglich kann die Expression des *MFα1*-Gens im Reporterstamm nicht induziert werden. Anhand dieser Kontrolle kann eine α-Faktor-unabhängige Ausbildung des Shmoo-Phänotyps ausgeschlossen werden.

Um ein Auftreten des Shmoo-Phänotyps unabhängig von der Interaktion zwischen Androgenrezeptor und Androgen zu überprüfen, werden die Hefestämme BY4741 *MAT*a p426*PGK-MFα1*, *p423GPD* (Sensorstamm) und BY4741 *MAT*a p426*FIG1**-MFα1*, p423*GPD* (Reporterstamm) miteinander kultiviert. In diesem Ansatz liegt kein Androgenrezeptor vor. Somit kann die Expression des *MFα1*-Gens weder im Sensor-, noch im Reporterstamm induziert werden. Für das Verstärkersystem werden die Hefestämme BY4741 *MAT*a p423*GPD-AR1*, p426*PGK-MFα1* und BY4741 *MAT*a p426*FIG1**-MFα1*, p423*GPD* in Anwesenheit verschiedener Konzentrationen einer androgen-wirksamen Substanz miteinander inkubiert.

Die Auswertung des Versuchs erfolgt, wie oben beschrieben. Die Kultivierung der Teststämme sowohl im Einzellsystem als auch im auf zwei unterschiedlichen Zelltypen basierenden Verstärkersystem in Anwesenheit von DHT führte zu einer lichtmikroskopisch beobachtbaren Ausbildung des Shmoo-Phänotyps.

### In der Erfindungsbeschreibung zitierte Nicht-Patentliteratur:

Ballensiefen W. und Schmitt H. D. (1997) Periplasmic Bar1 protease of Saccharomyces cerevisiae is active before reaching its extracellular destination. Eur. J. Biochem. 247: 142-147
Barkai N., Rose M. D. und Wingreen N. S. (1998) Protease helps yeast find mating partners. Nature 396: 422-423.
Bleve G., Lezzi C., Mita G., Rampino P., Perotta C., Villanova L. und Grieco F. (2008) Molecular cloning and heterologous expression of a laccase gene from Pleurotus eryngii in free and immobilized Saccharomyces cerevisiae cells. Appl. Microbiol. Biotechnol. 79: 731-741.
Bovee, T. F. H., Lommerse J. P., Peijnenburg A. A., Fernandes E. A. und Nielen M. W. (2008) A new highly androgen specific yeast biosensor, enabling optimisation of (Q)SAR model approaches. J. Steroid. Biochem. Mol. Biol. 108: 121-131.
Chan R. K. und Otte C. A. (1982) Physiological characterization of Saccharomyces cerevisiae mutants supersensitive to G1 arrest by a factor and alpha factor pheromones. Mol. Cell. Biol. 2: 21-29.
Chen, J. Ding M. und Pederson D. S. (1994) Binding of TFIID to the yeast CYC1 TATA boxes in yeast occurs independently of upstream activating sequences. Proc. Natl. Acad. Sci. USA 91: 11909-11913.
Chernaik M. L. und Huang P. C. (1991) Differential effect of cysteine-to-serine substitutions in metallothionein on cadmium resistance. Proc. Natl. Acad. Sci. USA 88: 3024-3028.
Chow C. M., Yagüe E., Raguz S., Wood D. A. und Thurston C. F. (1994) The cel3 gene of Agaricus bisporus codes for a modular cellulase and is transcriptionally regulated by the carbon source. Appl. Environ. Microbiol. 60: 2779-2785.
Doesburg P., Kuil C. W., Berrevoets C. A., Steketee K., Faber P. W., Mulder E., Brinkmann A. O. und Trapman J. (1997) Functional in vivo interaction between the amino-terminal, transactivation domain and the ligand binding domain of the androgen receptor. Biochemistry 36: 1052-1064.
Dolan J. W, Kirkman C. und Fields S. (1989) The yeast STE12 protein binds to the DNA sequence mediating pheromone induction. Proc. Natl. Acad. Sci. USA 86: 5703-5707.
Dolan J. W. und Fields S. (1990) Overproduction of the yeast STE12 protein leads to constitutive transcriptional induction. Genes Dev. 4: 492-502.
Dubbink H. J., Hersmus R., Verma C. S., van der Korput H. A., Berrevoets C. A., van Tol J., Ziel-van der Made A. C., Brinkmann A. O., Pike A. C. und Trapman J. (2004) Distinct recognition modes of FXXLF and LXXLL motifs by the androgen receptor. Mol. Endocrinol. 18: 2132-2150.
Ecker D. J., Butt T. R., Sternberg E. J., Neeper M. P., Debouck C., Gorman J. A. und Crooke S. T. (1986) Yeast metallothionein function in metal ion detoxification. J. Biol. Chem. 261: 16895-16900.
Erdman, S., Lin, L., Malczynski, M. und Snyder, M. (1998) Pheromone-regulated genes required for yeast mating differentiation. J. Cell Biol. 140: 461-483.
Fairman C., Zhou X.-L. und Kung C. (1999) Potassium uptake through the TOK1 K+ channel in the budding yeast. J. Membr. Biol. 168: 149-157.
Fine T., Leskinen P., Isobe T., Shiraishi H., Morita M., Marks R. S. und Virta M. (2006) Luminescent yeast cells entrapped in hydrogels for estrogenic endocrine disrupting chemical detection. Biosens. Bioelectron. 21: 2263-2269.
Hagen D. C., McCaffrey G., Sprague G. F. Jr. (1991) Pheromone response elements are necessary and sufficient for basal and pheromone-induced transcription of the FUS1 gene of Saccharomyces cerevisiae. Mol. Cell. Biol. 11: 2952-2961.
Harbison C. T., Gordon D. B., Lee T. I., Rinaldi N. J., Macisaac K. D., Danford T. W., Hannett N. M., Tagne J. B., Reynolds D. B., Yoo J., Jennings E. G., Zeitlinger J., Pokholok D. K., Kellis M., Rolfe P. A., Takusagawa K. T., Lander E. S., Gifford D. K., Fraenkel E. und Young R. A. (2004) Transcriptional regulatory code of a eukaryotic genome. Nature 431: 99-104.
Hentges P., Van Driessche B., Tafforeau L., Vandenhaute J., und Carr A. M. (2005) Three novel antibiotic marker cassettes for gene disruption and marker switching in Schizosaccharomyces pombe. Yeast 22: 1013-1019.
Hill J. E., Myers A. M., Koerner T. J. und Tzagoloff A. (1986) Yeast/E. coli shuttle vectors with multiple unique restriction sites. Yeast 2: 163-167.
Hottiger T., Fürst P., Pohlig G. und Heim J. (1994) Physiological characterization of the yeast metallothionein (CUP1) promoter, and consequences of overexpressing its transcriptional activator, ACEl. Yeast 10: 283-296.
Hwang-Shum J. J., Hagen D. C., Jarvis E. E., Westby C. A. und Sprague G. F. Jr. (1991). Relative contributions of MCM1 and STE12 to transcriptional activation of a- and α- specific genes from Saccharomyces cerevisiae. Mol. Gen. Genet. 227: 197-204.
Jackson C. L., Konopka J. B. und Hartwell L. H. (1991) S. cerevisiae α pheromone receptors activate a novel signal transduction pathway for mating partner discrimination. Cell 67: 389-402.
Janes M., Meyhack B., Zimmermann W. und Hinnen A. (1990) The influence of GAP promoter variants on hirudin production, average copy number and cell growth in Saccharomyces cerevisiae. Curr. Genet. 18: 97-103.
Jenster G., van der Korput H. A., Trapman J. und Brinkmann A. O. (1995) Identification of two transcription activation units in the N-terminal domain of the human androgen receptor. J. Biol. Chem. 270: 7341-7346.
Kaiser C., Michaelis S. und Mitchell A. (1994) Methods in Yeast Genetics. New York, Cold Spring Harbor Laboratory Press*.*
Kronstad J. W., Holly J. A. und MacKay V. L. (1987) A yeast operator overlaps an upstream activation site. Cell 50: 369-377.
Langley E., Zhou Z. X. und Wilson E. M. (1995) Evidence for an anti-parallel orientation of the ligand-activated human androgen receptor dimer. J. Biol. Chem. 270: 29983-29990.
Leberer E., Thomas D. Y. und Whiteway M. (1997) Pheromone signaling and polarized morphogenesis in yeast. Curr. Opin. Genet. Dev. 7: 59-66.
Mackay V. und Manney T. R. (1974) Mutations affecting sexual conjugation and related processes in Saccharomyces cerevisiae. I. Isolation and phenotypic characterization of nonmating mutants. Genetics 76: 255-271.
Marsh, L. and Rose, M.D. (1997) The pathway of cell and nuclear fusion during mating in S. cerevisiae. In: Pringle, J.R., Broach, J.R. and Jones, E.W. (eds.) The molecular and cellular biology of the yeast Saccharomyces. Cold Spring Laboratory press, New York.
McNabb D. S., Reed R. und Marciniak R. A. (2005) Dual luciferase assay system for rapid assessment of gene expression in Saccharomyces cerevisiae. Eukaryot. Cell 4: 1539-1549.
Mille Y., Girard J.-P., Beney L. und Gervais P. (2005) Air drying optimization of Saccharomyces cerevisiae through ist water-glycerol dehydration properties. J. Appl. Microbiol. 99: 376-382
Mooradian A. D., Morley J. E. und Korenman S. G. (1987) Biological actions of androgens. Endocr. Rev. 8: 1-28.
Muller, E. M., Mackin, N. A., Erdman, S. und Cunningham, K. W. (2003) Fig1p facilitates Ca2+ influx and cell fusion during mating of Saccharomyces cerevisiae. J. Biol. Chem. 278: 38461-38469.
Mumberg D., Mailer R. und Funk M. (1995) Yeast vectors for the controlled expression of heterologous proteins in different genetic backgrounds. Gene 156: 119-122.
Opekarova M., Caspari T., Pinson B., Brethes D. und Tanner W. (1998) Posttranslational fate of CAN1 permease of Saccharomyces cerevisiae. Yeast 14: 215-224.
Perez-Castineira J. R., Lopez-Marques R. L., Villalba J. M., Losada M. und Serrano A. (2002) Functional complementation of yeast cytosolic pyrophosphatase by bacterial and plant H+-translocating pyrophosphatases. Proc. Natl. Acad. Sci. USA 99: 15914-15919.
Protchenko O., Shakoury-Elizeh M., Keane P., Storey J., Androphy R. und Philpott C. C. (2008) Role of PUR1 in inducible porphyrin and heme transport in Saccharomyces cerevisiae. Eukaryot. Cell 7: 859-871.
Purvis I. J., Chotai D., Dykes C. W., Lubahn D. B., French F. S., Wilson E. M. und Hobden A. N. (1991) An androgen-inducible expression system for Saccharomyces cerevisiae. Gene 106: 35-42.
Roberts C. J., Nelson B., Marton M. J., Szoughton R., Meyer M. R., Bennett H. A., He Y. D., Dai H., Walker W. L., Hughes T. R., Tyers M., Boone C. und Friend S. H. (2000) Signaling and circuitry of multiple MAPK pathways revealed by a matrix of global gene expression profiles. Science 287: 873-880.
Routledge E. J. und Sumpter J. P. (1996) Estrogenic activity of surfactants and some of their degradation products assessed using a recombinant yeast screen. Environ. Toxicol. Chem. 15: 241-248.
Roy A. K., Lavrovsky Y., Song C. S., Chen S., Jung M. H., Velu N. K., Bi B. Y. und Chatterjee B.(1999) Regulation of androgen action. Vitam. Horm. 55: 309-352.
Segall J. E. (1993) Polarization of yeast cells in spatial gradients of α-mating factor. Proc. Natl. Acad. Sci. USA 90: 8332-8336.
Sekler I., Kopito R. und Casey J. R. (1995) High level expression, partial purification, and functional reconstitution of the human AE1 anion exchanger in Saccharomyces cerevisiae. J. Biol. Chem. 270: 21028-21034.
Sohoni P. und Sumpter J. P. (1998) Several environmental oestrogens are also antiandrogens. J. Endocrinol. 158: 327-339.
Sprague G. F. Jr und Herskowitz I. (1981) Control of yeast cell type by the mating type locus. I. Identification and control of expression of the a-specific gene BAR1. J. Mol. Biol. 153: 305-321.
Tkacz J. S. und MacKay V. L. (1979) Sexual conjugation in yeast: cell surface changes in response to the action of mating hormones. J. Cell Biol. 80: 326-333.
Wilson C. M. und McPhaul M. J. (1994) A and B forms of the androgen receptor are present in human genital skin fibroblasts. Proc. Natl. Acad. Sci. USA 91: 1234-1238.
Zhang, N.-N., Dudgeon D. D., Paliwal S., Levchenko A., Grote E. und Cunningham K. W. (2006) Multiple signaling pathways regulate yeast cell death during response to mating pheromones. Mol. Biol. Cell 17: 3409-3422.

## Patentansprüche

1. Verfahren zum Nachweis und/oder zur Identifizierung von hormonell wirksamen Substanzen, umfassend die folgenden Schritte:
a) eine Probe, die mutmaßlich eine hormonell wirksame Substanz umfasst, wird mit Hefekulturmedium oder Puffer versetzt,
b) die mit dem Hefekulturmedium oder Puffer versetzte Probe wird mit haploiden Hefezellen eines einheitlichen Typs kontaktiert, die die folgenden Merkmale 1 bis 3 umfassen:
1. die kodierende DNA-Sequenz für einen heterologen, nukleären Hormonrezeptor ist unter die Kontrolle eines konstitutiven Promotors gestellt und wird funktionell exprimiert,
2. die für ein Hefe-Pheromon kodierende DNA-Sequenz ist unter die Kontrolle eines durch den heterologen, nukleären Hormonrezeptor regulierbaren Promotors gestellt,
3. ein Gen, das für einen Rezeptor für das Hefe-Pheromon kodiert, wird konstitutiv und funktionell exprimiert,
wobei die Aktivierung des heterologen, nukleären Hormonrezeptors durch die hormonell wirksame Substanz zu der Expression der für das Hefe-Pheromon kodierenden DNA-Sequenz und zu der Sekretion des Pheromons aus der haploiden Hefezelle führt, und
c) ein gerichtetes Zellwandwachstum und/oder die Bildung von Zygoten von haploiden Hefezellen werden erfasst,
wobei die haploiden Hefezellen *Saccharomyces cerevisiae* oder *Schizosaccharomyces pombe* Zellen sind.

2. Verfahren gemäß einem der Ansprüche 1, **dadurch gekennzeichnet, dass** das gerichtete Zellwandwachstum und/oder die Bildung von Zygoten lichtmikroskopisch detektiert werden.

3. Verfahren gemäß Anspruch 1, **dadurch gekennzeichnet, dass** das gerichtete Zellwandwachstum und/oder die Bildung von Zygoten durch physikalische Messung der Trübung detektiert werden.

4. Verfahren gemäß einem der Ansprüche 1 bis 3, wobei zusätzlich zu dem gerichteten Zellwandwachstum und/oder der Bildung von Zygoten physiologische Änderungen von haploiden Hefezellen erfasst werden.

5. Verfahren gemäß Anspruch 4, **dadurch gekennzeichnet, dass** die physiologischen Änderungen der haploiden Hefezellen die Expression eines spezifischen Gens umfassen.

6. Verfahren gemäß Anspruch 5, **dadurch gekennzeichnet, dass** das spezifische Gen für ein Markerprotein kodiert.

7. Verfahren gemäß einem der Ansprüche 5 oder 6, **dadurch gekennzeichnet, dass** das spezifische Gen für ein fluoreszierendes Protein kodiert.

8. Verfahren gemäß einem der Ansprüche 1 bis 7, **dadurch gekennzeichnet, dass** der nukleäre Hormonrezeptor ausgewählt ist aus Androgenrezeptoren und Estrogenrezeptoren.

9. Haploide Hefezellen eines einheitlichen Typs mit den folgenden Merkmalen:
1. die kodierende DNA-Sequenz für einen heterologen, nukleären Hormonrezeptor ist unter die Kontrolle eines konstitutiven Promotors gestellt und wird funktionell exprimiert,
2. die für ein Hefe-Pheromon kodierende DNA-Sequenz ist unter die Kontrolle eines durch den heterologen, nukleären Hormonrezeptor regulierbaren Promotors gestellt,
wobei die Aktivierung des heterologen, nukleären Hormonrezeptors durch die hormonell wirksame Substanz zu der Expression der für das Hefe-Pheromon kodierenden DNA-Sequenz und zu der Sekretion des Pheromons aus der haploiden Hefezelle führt, **dadurch gekennzeichnet, dass** sie ein Gen, das für einen Rezeptor für das Hefe-Pheromon kodiert, konstitutiv und funktionell exprimieren und wobei die haploiden Hefezellen *Saccharomyces cerevisiae* oder *Schizosaccharomyces pombe* Zellen sind.

10. Verwendung des Verfahrens gemäß einem der Ansprüche 1 bis 8 oder der haploiden Hefezellen gemäß Anspruch 9 zum Nachweis von hormonell wirksamen Substanzen.

11. Verwendung gemäß Anspruch 10 zum Nachweis von hormonell wirksamen Substanzen in Urin-, Blut- und Serumproben.

12. Verwendung gemäß einem der Ansprüche 10 oder 11 zum Nachweis von Dopingmitteln in Urinproben.

13. Verwendung des Verfahrens gemäß einem der Ansprüche 1 bis 8 oder der haploiden Hefezellen gemäß Anspruch 9 zur Abschätzung der Belastung von Umweltproben mit hormonell wirksamen Substanzen.

14. Verwendung des Verfahrens gemäß einem der Ansprüche 1 bis 8 oder der haploiden Hefezellen gemäß Anspruch 9 für die Identifizierung potentiell hormonell wirksamer Stoffe für die pharmazeutische Anwendung.

## Claims

1. Method for verifying and/or identifying hormonally effective substances, comprising the steps of:
a) to a sample that comprises presumably a hormonally active substance a yeast culturing medium or buffer is added,
b) the sample to which the yeast culturing medium or buffer has been added is contacted with haploid yeast cells of a single type that comprise the following features 1 to 3:
1. the DNA sequence that codes for a heterologous, nuclear hormone receptor is subjected to the control of a constitutive promoter and is functionally expressed;
2. the DNA sequence coding for a yeast pheromone is subjected to the control of a promoter that can be regulated by the heterologous, nuclear hormone receptor;
3. a gene that codes for a receptor for the yeast pheromone is constitutively and functionally expressed,
wherein the activation of the heterologous, nuclear hormone receptor by the hormonally active substance causes the expression of the DNA sequence coding for the yeast pheromone and the secretion of the pheromone from the haploid yeast cell; and
c) a directed cell wall growth and/or formation of zygotes from haploid yeast cells are detected.
wherein the haploid yeast cells are *Saccharomyces cerevisiae* or *Schizosaccharomyces pompe* cells.

2. Method according to claim 1, **characterized in that** the directed cell wall growth and/or formation of zygotes are detected by a light microscope.

3. Method according to claim 1, **characterized in that** the directed cell wall growth and/or formation of zygotes are detected by physical measurement of the turbidity.

4. Method according to one of the claims 1 to 3, wherein additional to the directed cell wall growth and/or formation of zygotes physiological changes of the haploid yeast cells are detected.

5. Method according to claim 4, **characterized in that** physiological changes of the haploid yeast cells comprise the expression of a specific gene.

6. Method according to claim 5, **characterized in that** the specific gene codes for a marker protein.

7. Method according to one of the claims 5 or 6, **characterized in that** the specific gene codes for a fluorescent protein.

8. Method according to one of the claims 1 to 7, **characterized in that** the nuclear hormone receptor is selected from androgen receptors and estrogen receptors.

9. Haploid yeast cells of a single type having the following features:
1. the DNA sequence that codes for a heterologous, nuclear hormone receptor is subjected to the control of a constitutive promoter and is functionally expressed;
2. the DNA sequence coding for a yeast pheromone is subjected to the control of a promoter that can be regulated by the heterologous, nuclear hormone receptor;
wherein the activation of the heterologous, nuclear hormone receptor by the hormonally active substance causes the expression of the DNA sequence coding for the yeast pheromone and the secretion of the pheromone from the haploid yeast cell, **characterized in that** they constitutively and functionally express a gene that codes for a receptor of the yeast pheromone and wherein the haploid yeast cells are *Saccharomyces cerevisiae* or *Schizosaccharomyces pompe* cells.

10. Use of the method according to one of the claims 1 to 8 or of the haploid yeast cells according to claim 9 for detecting hormonally active substances.

11. Use according to claim 10 for detecting hormonally active substances in urine, blood, and serum samples.

12. Use according to one of the claims 10 or 11 for detecting doping agents in urine samples.

13. Use of the method according to one of the claims 1 to 8 or of the haploid yeast cells according to claim 9 for evaluating loading of environmental samples with hormonally active substances.

14. Use of the method according to one of the claims 1 to 8 or of the haploid yeast cells according to claim 9 for identifying potentially hormonally active substances for pharmaceutical application.

## Revendications

1. Procédé destiné à déceler et/ou à identifier des substances à activité hormonale, comprenant les étapes suivantes :
a) on mélange avec un milieu de culture de levure ou avec un tampon un échantillon qui comporte probablement une substance à activité hormonale,
b) on met en contact l'échantillon mélangé au milieu de culture de levure ou au tampon avec des cellules de levure haploïdes d'un type homogène, qui ont les caractéristiques 1 à 3 suivantes :
1. la séquence ADN codée pour un récepteur hormonal nucléaire hétérologue est placée sous le contrôle d'un promoteur constitutif et est fonctionnellement exprimée,
2. la séquence ADN codée pour un phéromone de levure est placée sous le contrôle d'un promoteur régulable par le récepteur hormonal nucléaire hétérologue,
3. un gène qui code pour un récepteur pour le phéromone de levure est constitutivement et fonctionnellement exprimé,
l'activation du récepteur hormonal nucléaire hétérologue par la substance à activité hormonale menant à l'expression de la séquence ADN codant pour le phéromone de levure et à la sécrétion du phéromone à partir de la cellule de levure haploïde et
c) on recense une croissance dirigée de la paroi des cellules et/ou la formation de zygotes de cellules de levure haploïdes,
les cellules de levure haploïdes étant des cellules *Saccharomyces cerevisiae* ou *Schizosaccharomyces pombe.*

2. Procédé selon la revendication 1, **caractérisé en ce qu'**on détecte par microscopie optique la croissance dirigée de la paroi des cellules et/ou la formation de zygotes.

3. Procédé selon la revendication 1, **caractérisé en ce qu'**on détecte par mesure physique de la turbidité la croissance dirigée de la paroi des cellules et/ou la formation de zygotes.

4. Procédé selon l'une quelconque des revendications 1 à 3, lors duquel, en supplément de la croissance dirigée de la paroi des cellules et/ou de la formation de zygotes, on recense des changements physiologiques de cellules de levure haploïdes.

5. Procédé selon la revendication 4, **caractérisé en ce que** les changements physiologiques des cellules de levure haploïdes englobent l'expression d'un gène spécifique.

6. Procédé selon la revendication 5 **caractérisé en ce que** le gène spécifique code pour une protéine marqueur.

7. Procédé selon l'une quelconque des revendications 5 ou 6, **caractérisé en ce que** le gène spécifique code pour une protéine fluorescente.

8. Procédé selon l'une quelconque des revendications 1 à 7, **caractérisé en ce que** le récepteur hormonal nucléaire est choisi parmi des récepteurs androgènes et des récepteurs estrogènes.

9. Cellules de levure haploïdes d'un type homogène, présentant les caractéristiques suivantes :
1. la séquence ADN codée pour un récepteur hormonal nucléaire hétérologue est placée sous le contrôle d'un promoteur constitutif et est fonctionnellement exprimée,
2. la séquence ADN codée pour un phéromone de levure est placée sous le contrôle d'un promoteur régulable par le récepteur hormonal nucléaire hétérologue,
3. un gène qui code pour un récepteur pour le phéromone de levure est constitutivement et fonctionnellement exprimé,
l'activation du récepteur hormonal nucléaire hétérologue par la substance à activité hormonale menant à l'expression de la séquence ADN codant pour le phéromone de levure et à la sécrétion du phéromone à partir de la cellule de levure haploïde, caractérisées qu'elles expriment constitutivement et fonctionnellement un gène qui code pour un récepteur pour le phéromone de levure et les cellules de levure haploïdes étant des cellules *Saccharomyces cerevisiae* ou *Schizosaccharomyces pombe.*

10. Utilisation du procédé selon l'une quelconque des revendications 1 à 8 ou des cellules de levure haploïdes selon la revendication 9 pour déceler des substances à activité hormonale.

11. Utilisation selon la revendication 10 pour déceler des substances à activité hormonale dans des échantillons d'urine, de sang et de sérum.

12. Utilisation selon l'une quelconque des revendications 10 ou 11 pour déceler des produits dopants dans des échantillons d'urine.

13. Utilisation du procédé selon l'une quelconque des revendications 1 à 8 ou des cellules de levure haploïdes selon la revendication 9 pour estimer la charge d'échantillons environnementaux en substances à activité hormonale.

14. Utilisation du procédé selon l'une quelconque des revendications 1 à 8 ou des cellules de levure haploïdes selon la revendication 9 pour l'identification de matières à activité potentiellement hormonale pour l'application pharmaceutique.


### SEQUENCE LISTING

<110> Technische Universität Dresden
<120> Verfahren zum Nachweis und/oder zur Identifzierung hormonell wirksamer Substanzen
<130> 00017P0124DE
<160> 19
<170> PatentIn version 3.5
<210> 1
   <211> 633
   <212> DNA
   <213> Artificial Sequence
<220>
   <223> Derivat des PGK-Promotors
<400> 1
   aacgagtgtt tccctccttc ttgaattgat gttaccctca taaagcacgt ggcctcttat      60
   cgagaaagaa attaccgtcg ctcgtgattt gtttgcaaaa agaacaaaac tgaaaaaacc     120
   ccggatcggt acaaaatgtt ctaggtacaa aatgttctcg gtacaaaatg ttctgagctc     180
   aaagcggccg cgatccggtc gtcacacaac aaggtcctag cgacggctca caggttttgt     240
   aacaagcaat cgaaggttct ggaatggcgg gaaagggttt agtaccacat gctatgatgc     300
   ccactgtgat ctccagagca aagttcgttc gatcgtactg ttactctctc tctttcaaac     360
   agaattgtcc gaatcgtgtg acaacaacag cctgttctca cacactcttt tcttctaacc     420
   aagggggtgg tttagtttag tagaacctcg tgaaacttac atttacatat atataaactt     480
   gcataaattg gtcaatgcaa gaaatacata tttggtcttt tctaattcgt agtttttcaa     540
   gttcttagat gctttctttt tctctttttt acagatcatc aaggaagtaa ttatctactt     600
   tttacaacaa atacaaaaga tctgctagca aaa                                   633
<210> 2
   <211> 38
   <212> DNA
   <213> Artificial Sequence
<220>
   <223> Primer
<400> 2
   tattatgagc tcttgaatga tcaaccaaac gccgatat 38
<210> 3
   <211> 57
   <212> DNA
   <213> Artificial Sequence
<220>
   <223> Primer
<400> 3
   tattatacta gttttttttt tttttttttt gtttgtttgt ttgtttgttt actataa 57
<210> 4
   <211> 537
   <212> DNA
   <213> Artificial Sequence
<220>
   <223> Kodierende Sequenz der 178 carboxyterminalen Aminosäuren von Cln2p
<220>
   <221> CDS
   <222> (1)..(537)
<400> 4
   gca tcc aac ttg aac att tcg aga aag ctt acc ata tca acc cca tca        48
   Ala Ser Asn Leu Asn Ile Ser Arg Lys Leu Thr Ile Ser Thr Pro Ser
   1               5                   10                  15
   tgc tct ttc gaa aat tca aat agc aca tcc att cct tcg ccc gct tcc        96
   Cys Ser Phe Glu Asn Ser Asn Ser Thr Ser Ile Pro Ser Pro Ala Ser
               20                  25                  30
   tca tct caa agc cac act cca atg aga aac atg agc tca ctc tct gat       144
   Ser Ser Gln Ser His Thr Pro Met Arg Asn Met Ser Ser Leu Ser Asp
           35                  40                  45
   aac agc gtt ttc agc cgg aat atg gaa caa tca tca cca atc act cca       192
   Asn Ser Val Phe Ser Arg Asn Met Glu Gln Ser Ser Pro Ile Thr Pro
       50                  55                  60
   agt atg tac caa ttt ggt cag cag cag tca aac agt ata tgt ggt agc       240
   Ser Met Tyr Gln Phe Gly Gln Gln Gln Ser Asn Ser Ile Cys Gly Ser
   65                  70                  75                  80
   acc gtt agt gtg aat agt ctg gtg aat aca aat aac aaa caa agg atc       288
   Thr Val Ser Val Asn Ser Leu Val Asn Thr Asn Asn Lys Gln Arg Ile
                   85                  90                  95
   tac gaa caa atc acg ggt cct aac agc aat aac gca acc aat gat tat       336
   Tyr Glu Gln Ile Thr Gly Pro Asn Ser Asn Asn Ala Thr Asn Asp Tyr
               100                 105                 110
   att gat ttg cta aac cta aat gag tct aac aag gaa aac caa aat ccc       384
   Ile Asp Leu Leu Asn Leu Asn Glu Ser Asn Lys Glu Asn Gln Asn Pro
           115                 120                 125
   gca acg gcg cat tac ctc aat ggg ggc cca ccc aag aca agc ttc att       432
   Ala Thr Ala His Tyr Leu Asn Gly Gly Pro Pro Lys Thr Ser Phe Ile
       130                 135                 140
   aac cat gga atg ttc ccc tcg cca act ggg acc ata aat agc ggt aaa       480
   Asn His Gly Met Phe Pro Ser Pro Thr Gly Thr Ile Asn Ser Gly Lys
   145                 150                 155                 160
   tct agc agt gcc tca tct tta att tct ttt ggt atg ggc aat acc caa       528
   Ser Ser Ser Ala Ser Ser Leu Ile Ser Phe Gly Met Gly Asn Thr Gln
                   165                 170                 175
   gta ata tag                                                           537
   Val Ile
<210> 5
   <211> 178
   <212> PRT
   <213> Artificial Sequence
<220>
   <223> 178 carboxyterminale Aminosäuren von Cln2p
<400> 5
   Ala Ser Asn Leu Asn Ile Ser Arg Lys Leu Thr Ile Ser Thr Pro Ser
   1               5                   10                  15
   Cys Ser Phe Glu Asn Ser Asn Ser Thr Ser Ile Pro Ser Pro Ala Ser
               20                  25                  30
   Ser Ser Gln Ser His Thr Pro Met Arg Asn Met Ser Ser Leu Ser Asp
           35                  40                  45
   Asn Ser Val Phe Ser Arg Asn Met Glu Gln Ser Ser Pro Ile Thr Pro
       50                  55                  60
   Ser Met Tyr Gln Phe Gly Gln Gln Gln Ser Asn Ser Ile Cys Gly Ser
   65                  70                  75                  80
   Thr Val Ser Val Asn Ser Leu Val Asn Thr Asn Asn Lys Gln Arg Ile
                   85                  90                  95
   Tyr Glu Gln Ile Thr Gly Pro Asn Ser Asn Asn Ala Thr Asn Asp Tyr
               100                 105                 110
   Ile Asp Leu Leu Asn Leu Asn Glu Ser Asn Lys Glu Asn Gln Asn Pro
           115                 120                 125
   Ala Thr Ala His Tyr Leu Asn Gly Gly Pro Pro Lys Thr Ser Phe Ile
       130                 135                 140
   Asn His Gly Met Phe Pro Ser Pro Thr Gly Thr Ile Asn Ser Gly Lys
   145                 150                 155                 160
   Ser Ser Ser Ala Ser Ser Leu Ile Ser Phe Gly Met Gly Asn Thr Gln
                   165                 170                 175
   Val Ile
<210> 6
   <211> 32
   <212> DNA
   <213> Artificial Sequence
<220>
   <223> Primer
<400> 6
   tatataacta gtatggaagt gcagttaggg ct 32
<210> 7
   <211> 34
   <212> DNA
   <213> Artificial Sequence
<220>
   <223> Primer
<400> 7
   atatatgtcg actcactggg tgtggaaata gatg 34
<210> 8
   <211> 24
   <212> DNA
   <213> Artificial Sequence
<220>
   <223> Primer
<400> 8
   cggtaggtat tgattgtaat tctg 24
<210> 9
   <211> 37
   <212> DNA
   <213> Artificial Sequence
<220>
   <223> Primer
<400> 9
   tatattacta gtatgagatt tccttcaatt tttactg 37
<210> 10
   <211> 31
   <212> DNA
   <213> Artificial Sequence
<220>
   <223> Primer
<400> 10
   tatattggat ccttagtaca ttggttggcc g 31
<210> 11
   <211> 26
   <212> DNA
   <213> Artificial Sequence
<220>
   <223> Primer
<400> 11
   tcgtagtttt tcaagttctt agatgc 26
<210> 12
   <211> 70
   <212> DNA
   <213> Artificial Sequence
<220>
   <223> Primer
<400> 12
   aagaagatta caaactatca atttcataca caatataaac gattaaaaga cggatccccg 60
   ggttaattaa 70
<210> 13
   <211> 70
   <212> DNA
   <213> Artificial Sequence
<220>
   <223> Primer
<400> 13
   tgggaacaaa gtcgactttg ttacatctac actgttgtta tcagtcgggc gaattcgagc 60
   tcgtttaaac 70
<210> 14
   <211> 70
   <212> DNA
   <213> Artificial Sequence
<220>
   <223> TTACTACCATCACCTGCATCAAATTCCAGTAAATTCACATATTGGAGAAACGGATCCCCGGGTTA ATTAA
<400> 14
   ttactaccat cacctgcatc aaattccagt aaattcacat attggagaaa cggatccccg 60
   ggttaattaa 70
<210> 15
   <211> 70
   <212> DNA
   <213> Artificial Sequence
<220>
   <223> Primer
<400> 15
   atgaacgtga aagaaatcga gagggtttag aagtagttta gggtcatttt gaattcgagc 60
   tcgtttaaac 70
<210> 16
   <211> 70
   <212> DNA
   <213> Artificial Sequence
<220>
   <223> Primer
<400> 16
   atcgcctaaa atcataccaa aataaaaaga gtgtctagaa gggtcatata cggatccccg 60
   ggttaattaa 70
<210> 17
   <211> 70
   <212> DNA
   <213> Artificial Sequence
<220>
   <223> Primer
<400> 17
   actatatatt tgatatttat atgctataaa gaaattgtac tccagatttc gaattcgagc 60
   tcgtttaaac 70
<210> 18
   <211> 33
   <212> DNA
   <213> Artificial Sequence
<220>
   <223> Primer
<400> 18
   tattatgaat tcatggtgag caagggcgag gag 33
<210> 19
   <211> 37
   <212> DNA
   <213> Artificial Sequence
<220>
   <223> Primer
<400> 19
   tattatgtcg acttacttgt acagctcgtc catgccg 37